# EUROPEAN PATENT SPECIFICATION

(11) **EP 2 026 747 B1**
(45) Date of publication and mention of the grant of the patent: **27.01.2016**
(21) Application number: 07797762.7
(22) Date of filing: 25.05.2007
(51) Int. Cl.: A61K 8/11, A61K 8/66, A61Q 11/00

(54) **ORAL COMPOSITIONS PROVIDING ENHANCED TOOTH STAIN REMOVAL**
ORALE ZUSAMMENSETZUNGEN ZUR BEREITSTELLUNG EINER VERBESSERTEN ZAHNFLECKEN-ENTFERNUNG
COMPOSITIONS ORALES PERMETTANT D'ÉLIMINER EFFICACEMENT LES TACHES SUR LES DENTS

(30) Priority: 25.05.2006 US 803174 P
(43) Date of publication of application: 25.02.2009
(73) Proprietor: WM. WRIGLEY JR. COMPANY, Chicago, IL 60611 (US)
(72) Inventor: HAAS, Michael, S., Naperville, Illinois 60540 (US)
(74) Representative: Nash, David Allan
(86) International application number: PCT/US2007/069718
(87) International publication number: WO 2007/140286

(56) References cited:
- DE-B- 1 227 855
- GB-A- 1 255 284
- GB-A- 1 277 337
- US-A1- 2001 002 252
- US-A1- 2002 028 251

## Description

### FIELD OF THE INVENTION

This invention generally relates to oral compositions (e.g., a confection or chewing gum product such as a compressed chewing gum) effective for removal of stains from a tooth surface. In particular, this invention relates to oral compositions including a coated or an encapsulated protease enzyme to provide an oral composition effective for tooth stain removal.

### SUMMARY OF THE INVENTION

Briefly, therefore, the present invention is directed to an oral composition effective for stain removal from a tooth surface. The oral composition comprises an encapsulated protease enzyme having a moisture content of less than 2 wt-%, wherein the encapuslated protease enzyme comprises a protease enzyme effective for stain removal from a tooth surface and an encapsulant at least partially coating a surface of the protease enzyme.

The present invention is further directed to a method for preparing an oral composition effective for stain removal from a tooth surface. The method comprises contacting an encapsulated protease enzyme having a moisture content of less than 2 wt-%, with one or more components of the oral composition. The encapsulated protease enzyme comprises a protease enzyme effective for stain removal from a tooth surface and an encapsulant at least partially coating a surface of the enzyme.

### BRIEF DESCRIPTION OF THE DRAWINGS

Fig. 1 is a stain removal dose response plot for the testing of an enzyme (i.e., subtilisin) described in Example 42.
Fig. 2 provides a comparison of stain removal efficacy for an enzyme (i.e., subtilisin) and negative and positive controls as described in Example 42.
Fig. 3 is a plot of active (i.e., subtilisin) concentration versus sample time as described in Example 43.
Fig. 4 provides a comparison of in-vitro testing described in Example 42 and testing described in Example 43.

### DETAILED DESCRIPTION OF THE PREFERRED EMBODIMENTS

Protease enzymes can be used for stain removal from tooth surfaces by disrupting and/or removing the protein portion of one or more layers of the tooth (e.g., pellicle or plaque layer) and, accordingly, disrupting and/or removing the stain bound to these proteins. Unfortunately, many or most of the enzymes that might otherwise be effective for stain removal are not suitable or efficacious in various oral care compositions, because of their lack of stability, particularly their lack of thermal stability for use in high temperature processes (e.g., extruded gum processes). However, in accordance with the present invention, and as further detailed herein below, it has been discovered that oral compositions containing coated or encapsulated protease enzymes are effective for removal of stains from teeth and may be used in low temperature, low moisture content compositions such as compressed mint and gum formulations, as well as others noted elsewhere herein. The coated or encapsulated protease enzymes are efficacious for stain removal from teeth while also exhibiting improved stability and compatibility as compared to non-coated or non-encapsulated protease enzymes. More particularly, the coated or encapsulated protease enzymes exhibit improved stability under and compatibility with conditions typically used for the preparation of granular and/or low moisture content oral compositions of which they are a part.

In various embodiments, the coated or encapsulated enzymes may be incorporated into oral compositions that are prepared under relatively mild conditions that limit or prevent denaturing of the enzyme. In particular, the encapsulated enzyme may be incorporated into oral compositions that are prepared in a manner such that the temperature of the mixture of one or more ingredients of the oral composition before and/or after introduction of the encapsulated enzyme thereto is sufficiently low, such that denaturing of the enzyme is substantially limited, if not prevented. Additionally, the encapsulated enzyme is prepared in a manner such that the moisture content therein is sufficiently low for use in the desired oral composition, i.e. the moisture content of the encapsulated enzyme is less than 2 wt%, or even less than about 1 wt%. For example, in certain embodiments in which the encapsulated enzyme is prepared by spray drying, the moisture content of the encapsulated enzyme may be about 1.5 wt%.

In this regard it is to be noted that the process conditions (e.g., temperature, pH, moisture, etc.) for a given oral composition and encapsulated enzyme will be, at least in part, a function of the enzyme to be used, and the desired moisture content of the oral composition in which it is to be incorporated. Accordingly, an acceptable combination of encapsulated enzyme and oral composition (e.g., oral composition components, process conditions, etc.) may be determined using means known in the art.

It is to be further noted that improvements in stability of the coated or encapsulated protease enzymes may be measured or determined using means known in the art. For example, the stability of coated or encapsulated versus non-coated or non-encapsulated enzymes may be measured or determined using temperature activity profiles and/or temperature coefficients (e.g., the ratio of the rate of chemical reaction at one temperature (Vt) to the rate of the reaction at a temperature 10°C lower than the first).

Without being held to any particular theory, it is generally believed that protease enzymes act to disrupt and/or remove the protein portion of the pellicle/plaque layer that forms on the surface of teeth over time, thus removing the stains that are bound to these proteins. Protease enzymes, which may also be referred to as proteolytic enzymes or proteinases, in particular, are enzymes whose catalytic function is to hydrolyze or break down peptide bonds of these proteins. Protease enzymes differ in their ability to hydrolyze various peptide bonds; that is, each type of protease enzyme breaks a particular type of peptide bond. Protease enzymes suitable for use in accordance with the present invention include, for example, cathepsins, pepsin, rennin, thermolysin, trypsin, elastase, chymotrypsin, papain, bromelain, subtilisin, and combinations thereof. In various preferred embodiments, the protease enzyme comprises subtilisin. Protease enzymes suitable for use in accordance with the present invention may be characterized by their mode of action including, for example, as serine proteases, fungal proteases, and/or parasitic proteases. For example, chymotrypsin, trypsin, elastase, and subtilisin may be characterized as serine proteases. Protease enzymes suitable for use in the present invention may be derived from a variety of sources including plants, animal, and microbes. For example, papain and bromelain may be plant-derived (e.g., derived from plant sources such as pineapple, papaya, and kiwi), trypsin and chymotrypsin may be animal-derived, and subtilisin may be derived from, for example, A. niger and/or bacillus subtilis.

Generally, the pH of a solution containing an encapsulated enzyme (e.g., 1 wt% encapsulated enzyme in water) prepared in accordance with the present invention (e.g., by spray drying) may range from about 3 to about 10, from about 4 to about 9, or from about 5 to about 8. More particularly, it is currently believed that the pH of solutions containing encapsulated enzymes exhibiting relatively high activities, as detailed elsewhere herein, typically may exhibit a pH of from about 5 to about 8.

Further in accordance with these and other embodiments including, for example, encapsulated enzymes prepared by spray drying, the enzyme may typically contain a relatively low proportion of components that are not water soluble. These water insoluble components may include components derived from or part of the encapsulant (e.g., encapsulation matrix). More specifically, depending on the encapsulant used, these water insoluble components may include inorganic mineral compounds including, for example, calcium phosphate, talc, and/or calcium carbonate. For example, in certain embodiments the encapsulated enzyme may contain less than about 5 wt%, less than about 2.5 wt%, less than about 1.5 wt%, less than about 1 wt%, or less than about 0.5 wt% of water insoluble components.

### 1. Methods for Coating or Encapsulating

In accordance with the present invention, the protease enzyme may be coated (i.e., encapsulated) in a one-step or multi-step process using one or more coating or encapsulation methods generally known in the art, and further detailed herein below. In particular, the protease enzyme may be coated using a method, and a coating, generally known in the art, and as further detailed herein, in order to obtain a coated enzyme having improved processing capabilities, release properties, sensory properties, and/or stability. Coating or encapsulation methods known in the art, which are suitable for use in accordance with the present invention and which may be used alone or in combination, include for example spray drying, spray cooling, spray chilling, freeze-drying (i.e., lyophilizing), extrusion processes, coacervation, molecular inclusion, fluid bed coating, granulation, agglomeration, roll compaction, and absorbing the enzyme onto a support.

It is to be understood that reference to an encapsulated or coated protease enzyme herein generally refers to an enzyme that is at least partially coated or encapsulated by a coating composition (e.g., encapsulant). In one particular embodiment, substantially all the enzyme surface is coated by the encapsulant. In this regard it is to be further noted that, in those embodiments wherein the enzyme is not fully coated or encapsulated with a coating composition or encapsulant, a portion of the non-coated surface of the enzyme may be in direct contact with other ingredients or components of the oral composition including, for example, the gum base of a chewing gum composition. In such instances, it is to be understood that, in accordance with the present invention, this interaction or contact between the non-coated surface of the enzyme and other ingredients or components of the oral composition does not represent "encapsulation."

It is to be noted that essentially any encapsulation method known in the art may be used in accordance with the present invention, provided the method does not involve or utilize steps, conditions, etc. that act to denature or deactivate the enzyme to be encapsulated to any substantial degree. For example, due to the temperature sensitivity of the enzyme, the temperature of both the process to encapsulate and incorporate the enzyme into the oral composition may typically be less than 70°C, less than 60°C, less than 50°C, or even less than 40°C, depending upon the enzyme to be encapsulated. In one particular embodiment, the method employed is such that the resulting encapsulated or coated enzyme is at least about 25% active (i.e., at least about 25% of the enzyme's activity has been retained throughout the encapsulating/coating process), at least about 50% active, at least about 75% active, at least about 85% active, at least about 95% active or more.

It is to be further noted that, in addition to activity, the method of coating or encapsulation, and/or the steps or conditions used therein, may be controlled in order to optimize the size and/or control the release of the resulting encapsulated or coated enzyme. Accordingly, the particle size of the coated or encapsulated enzyme may, for example, be optimized for use in a chewing gum composition, compressed or tableted gum composition, mint, etc. Typically, however, the particle size of the coated or encapsulated enzyme may be at least about 10 microns, at least about 20 microns, or at least about 30 microns. In various embodiments including, for example, those in which the encapsulated enzyme is prepared by spray drying, a substantial portion of the encapsulated enzyme (e.g., at least about 90 wt%, or from 95 wt% to 100 wt%), exhibits a particle size of less than about 250 microns, less than about 200 microns, less than about 150 micron, or less than about 100 microns. Typically, the encapsulated protease enzyme exhibits a particle size of less than about 60 microns, less than 50 microns, or less than about 40 microns, the size for example falling within the range of about 10 to about 60 microns, about 20 to about 50 microns, or about 30 to about 40 microns. It is to be again further noted, however, that the particle size may be other than noted herein, without departing from the scope of the present invention.

Generally, regardless of the methods used to prepare the encapsulated enzyme, the encapsulated enzyme may typically have an enzyme concentration or, loading, of at least about 5 wt%, at least about 10 wt%, at least about 15 wt%, at least about 20 wt%, at least about 30 wt%, at least about 40 wt%, at least about 50 wt%, at least about 75 wt%, at least about 85 wt%, at least about 95 wt% (of the encapsulated protease enzyme), or more, the loading for example ranging from about 5 wt% to about 95 wt%, from about 10 wt% to about 85 wt%, from about 10 wt% to about 50 wt%, from about 15 wt% to about 50 wt%, from about 25 wt% to about 40 wt%, or from about 20 wt% to about 40 wt% (of the encapsulated protease enzyme).

Suitability of an encapsulant for use in accordance with the present invention will at least in part be a function of its compatibility with the enzyme to be encapsulated therewith, and/or its compatibility with the composition of which it is to be a part. Additionally, the encapsulant will generally exhibit organic solubility, good film-forming properties and low water solubility. Such encapsulants generally include, for example, acrylic polymers and copolymers, carboxyvinyl polymer, polyamides, polystyrene, polyvinyl acetate, polyvinyl acetate phthalate, polyvinylpyrrolidone, and waxes, as well as combinations thereof. Although all of these materials are possible for encapsulation of the enzyme(s), typically only food-grade materials are considered. Two standard food-grade coating materials that are good film formers and exhibit relatively low solubility in water are shellac and zein. Others which are slightly more water soluble, but still good film formers, are materials such as agar, alginates, a wide range of cellulose derivatives (such as for example ethyl cellulose, methyl cellulose, sodium hydroxymethyl cellulose, and hydroxypropylmethyl cellulose (HPMC)), dextrin, gelatin, and modified starches, as well as combinations thereof. Other encapsulants like acacia or maltodextrin are also suitable for use, alone or in combination with one or more acceptable encapsulants.

It is to be understood that the various encapsulants and encapsulation techniques known in the art, and/or as detailed herein, may be used alone, or in combination, in the manufacture of various chewing gum compositions, and/or the manufacture of various other oral compositions (e.g., confections).

One particular method for preparing an encapsulated protease enzyme includes spray drying in which a feed material (i.e., a mixture of the enzyme and encapsulant) is transformed from a fluid state into a dried particulate form by spraying the feed into a hot drying medium. Typically, spray drying is a one-step, continuous process in which the feed material is in the form of a solution, suspension, paste, or a combination thereof. The resulting dried product is generally in the form of powder, granule, and/or agglomerates, the precise form of the dried product depending, at least in part, on the physical and/or chemical properties of the feed, as well as the dryer design and operation. Spray drying generally includes atomization of a feed material (e.g., forming a spray of individual droplets from a bulk liquid), and contact between the spray and a drying medium (e.g., air) to result in evaporation of moisture from the feed material and encapsulation of the enzyme. Drying of the spray proceeds until a desired moisture content of the dried particles is obtained, and the product is then recovered from the air. Advantageously, spray drying can be conducted using means and equipment generally known in the art.

Encapsulating agents useful in the spray drying process may possess one or more of the following properties: they may be emulsifiers; they may be good film formers; they may have low viscosity at high solids levels (e.g., less than about 500 centipoise (cps) at greater than about 45% solids levels); they may have low hygroscopicity; they may release a flavor, sweetener, or an enzyme, when reconstituted in a finished food product; they may be inexpensive; they have little or no taste; and, they are typically commercially available. Hydrolyzed starches, modified starches, gum arabic, cellulose materials (e.g., hydroxypropylmethyl cellulose), and polyols are encapsulants typically used in spray drying. However, it should be understood that other encapsulants generally known in the art, and/or as detailed elsewhere herein, are likewise suitable for use in spray drying processes.

In one particular embodiment of the present invention, subtilisin may be spray-dried with an encapsulant comprising a cellulose material (e.g., hydroxylpropylcellulose, hydroxypropylmethyl cellulose, or another cellulose material noted elsewhere herein), the resulting encapsulated enzyme having, for example, about a 15 wt% loading, about a 20 wt% loading, or about a 25 wt% loading of the subtilisin enzyme (i.e., subtilisin constituting about 15 wt%, about 20 wt%, about 25 wt%, or about 25 wt% to about 40 wt% of the encapsulated enzyme). The encapsulated enzyme is well-suited for use in, for example, a chewing gum composition such as a compressed chewing gum.

Spray cooling and spray chilling encapsulation methods are also suitable for use in accordance with the present invention, and are similar to spray drying in that each of these methods involves dispersing the core material (i.e., the enzyme) into a liquefied coating material, or encapsulant, and spraying the mixture through a heated nozzle into a controlled environment. However, in contrast to spray drying that uses heated air to volatize the solvent from a coating dispersion, spray cooling and spray chilling use air cooled to ambient or refrigerated temperatures considerably below the solidification point of the encapsulant (e.g., a molten fat or wax). Spray cooling and spray chilling differ by the temperature of the air used to cool the encapsulant. For example, spray cooling typically uses air at temperatures of from greater than about 45°C to less than about 125°C. In at least one embodiment, spray cooling is conducted at a relatively low temperature (e.g., less than 70°C, less than 60°C, or less than 50°C) in order to limit, or even prevent, denaturation of the enzyme. Spray chilling typically uses air at a temperature of from about 25°C to about 40°C. Accordingly, for spray cooling and spray chilling, a wide variety of encapsulating materials having melting points falling within these noted temperature ranges may be employed. For example, for spray cooling, the encapsulant may be a vegetable oil or a derivative thereof, or alternatively a fat or a stearine, with melting points falling within the range of from about 45°C to about 120°C, as well as hard monoglycerides and diglycerides with melting points falling within the range of about 45°C to about 65°C. For spray chilling, the encapsulant may be, for example, a fractionated or hydrogenated vegetable oil with a comparatively lower melting point than the oils used in spray cooling (e.g., oils having, for example, melting points within the range of from about 30°C to about 40°C).

It is to be noted that, in one particular embodiment, subtilisin may be spray-cooled with an encapsulant comprising a vegetable oil, the resulting encapsulated enzyme having, for example, about a 35 wt% loading, about a 40 wt% loading, or about a 45 wt% loading of the subtilisin enzyme (i.e., subtilisin constituting about 35 wt%, about 40 wt% or about 45 wt% of the encapsulated enzyme). The encapsulated enzyme is well-suited for use in, for example, the coating layer of confectionery composition.

Freeze-drying methods suitable for encapsulating the enzyme in accordance with the present invention are generally known in the art and are described in, for example, U.S. Patent Nos. 5,031,336, 4,797,290, and 4,205,132, the entire contents of which are hereby incorporated by reference for all relevant purposes.

An additional method for encapsulating protease enzymes includes extrusion of a molten mass of the enzyme and encapsulant through a series of dies and into a dehydrating liquid, using means known in the art. More specifically, in accordance with this method, an encapsulant, such as a water soluble polymer, and an enzyme are preblended, prior to fiber extrusion. Alternatively, however, the enzyme may be added to the encapsulant after it has been melted. As the enzyme/encapsulant (e.g., polymer) mixture is extruded, small fibers are formed which, upon contacting the liquid, are cooled, the coating material (e.g., encapsulant) hardens or solidifies, forming an encapsulating matrix to entrap the enzyme. The extruded filaments are separated from the liquid bath, dried to limit hygroscopicity, and milled. This encapsulation method generally results in a longer, more delayed release of the active ingredient (e.g., enzyme) than other encapsulating methods noted herein (e.g., spray drying). A number of encapsulants may be used with this method including, for example, vinyl polymers such as polyvinyl alcohol or polyethylene, as well as other types of plastic polymers, and/or cellulose materials (e.g., hydroxypropylcellulose). This method of encapsulation is described in U.S. Patent No. 4,978,537.

Another extrusion method suitable for preparation of encapsulated protease enzymes in accordance with the present invention involves concentric coextrusion of the enzyme and encapsulant through a two-chambered nozzle. In particular, passage of the core material or, enzyme, through the inner nozzle and passage of the encapsulant through the outer nozzle, that results in the formation of a capsule or drop composed of a core fluid (e.g., enzyme) encapsulated by a layer or shell of the encapsulant. As the liquid stream exits the nozzle, local disturbances, such as induced vibration or gravitational, centrifugal, or drag force, control particle size. The shell is then hardened by appropriate means; for example, by cooling, chemical cross-linking, etc.

The size of the capsules, or encapsulated enzyme particles, produced, as well as the quantity of core material contained within each capsule, may depend on the physical properties of the fluids (e.g., densities, viscosities, and interfacial tensions), the processing conditions (e.g., flow rates and temperatures), the geometry of the nozzle (e.g., diameters of the inner and outer orifices), and/or the amplitude and frequency of small vibrational disturbances (natural or imposed) present in the system. Because there are so many variables, and because it is often difficult to vary one without affecting another (for example, changing the viscosity of the shell fluid changes the interfacial tension between it and the surrounding fluid, and between it and the core fluid), it is difficult to isolate the influence of the individual factors. For this reason, co-extrusion processes are typically designed, and operating conditions determined, on a case-by-case basis, using means known in the art.

It is to be noted that, in one particular embodiment, chymotrypsin is extruded in a melt with an encapsulant (e.g., a carbohydrate encapsulant such as sucrose, maltodextrin, or a combination thereof), the resulting encapsulated enzyme having, for example, about a 5 wt% loading, about a 10 wt% loading or about a 15 wt% loading of the chymotrypsin enzyme; that is, chymotripsin constitutes about 5 wt%, about 10 wt% or about 15 wt% of the encapsulated enzyme, and the encapsulating carbohydrate constitutes about 85 wt%, about 90 wt% or about 95 wt% of the encapsulated enzyme (e.g., about 45 wt% to about 55 wt% sucrose and about 35 wt% to about 45 wt%). The encapsulated enzyme is well suited for use in, for example, a compressed chewing gum composition.

Yet another method for encapsulating an enzyme involves coacervation. Coacervative encapsulation generally includes the steps of: (1) particle or droplet formation, (2) coacervative wall formation, and (3) capsule isolation. When two or more oppositely charged macromolecular colloids are used to form the coacervate, the process is termed complex coacervation. Colloids that have a positive charge include gelatin and agar; colloids that have a negative charge include carboxymethylcellulose and gum arabic. Depending upon the isoelectric point of each colloid, dilution with water and/or adjustment of pH may be necessary for the particular colloids to be oppositely charged. These reactions generally occur at a temperature above the gelling temperature for either colloid; otherwise, the colloids will not be in a liquid phase and coacervation will not occur. When coacervation occurs in an environment that contains oil particles, the oil particles act as nucleating agents and the protein colloids deposit as a shell-like structure around each oil particle. Proteins are typically used for coacervation whereby the coating material forms microcapsules around the material to be encapsulated, and is separated from the aqueous phase by further processing. Cross-linking of the protein shell of the complex coacervate may render the protein-encapsulated oil thermostable. The presence of crosslinks may also add greatly to the native protein structure and make the molecule resistant to unfolding or denaturation.

Molecular inclusion is another method for achieving encapsulation. This technique takes place at the molecular level and typically utilizes β-cyclodextrin, or a similar compound, as the encapsulating material. β-cyclodextrin generally has limited solubility, a hydrophobic center, and a relatively hydrophilic outer surface, all of which affect its ability to form complexes. The β-cyclodextrin molecule forms inclusion complexes with compounds that can fit dimensionally into its central cavity. These complexes are formed in a reaction that takes place in the presence of water. Molecules that are less polar than water, and those that have suitable dimensions to fit inside the cyclodextrin interior, can be included in the cyclodextrin molecule. The composition of the cyclodextrin complex depends on the molecular weight of the guest molecule. Because one molecule of cyclodextrin will normally include one guest molecule, the loading depends on the compounds included.

Another encapsulation method suitable for use in accordance with the present invention is fluidized bed processing, or, spray coating (often referred to as "air suspension coating"), which is generally conducted by suspending solid particles of the enzyme in an upward moving stream of air, which may be heated or cooled. The encapsulant, which may be in a molten state or dissolved in an evaporable solvent, is typically selected from among cellulose derivatives, dextrins, emulsifiers, lipids, protein derivatives, and starch derivatives. The coating is atomized and deposited on suspended particles. The turbulence of the column of air is sufficient to maintain suspension of the coated particles, allowing them to tumble and thereby become uniformly coated. Upon reaching the top of the air stream, the particles move into the outer, downward moving column of air that returns them to the fluidized bed with their coating nearly dried and hardened. The particles pass through the coating cycle many times per minute. With each successive pass, the random orientation of the particles further ensures their uniform coating.

Granulation or, agglomeration, methods may also be used in processes for preparing encapsulated protease enzymes. Granulation or agglomeration may be generally defined as any process in which relatively small particles are gathered into larger, permanent masses in which the original particles can still be identified. Granulation may be used to encapsulate enzymes to improve the flow properties and/or compression characteristics of the enzyme. Granulation may also be used to induce delayed release of the enzyme. The principal methods of granulating may be classified into three general categories: wet, dry, and other.

In a wet granulation method, a granulating liquid is used to facilitate the agglomeration process. The mixture is prepared in such a way as to have individual wet particles in contact with each other so that a partial coating can be applied. After the water or solvent is removed, the mixture may be ground and used as a powdered, coated product. Typically, wet massing of powders is carried out in high-shear mixers before wet screening, and often, the moist granulates are dried in fluidized bed equipment. Often, wet granulation is also carried out in fluid bed drier-granulators in which the liquid phase is sprayed onto fluidized powders as the hot airflow simultaneously dries the granules. There are primarily three ways in which powders can be increased in size in the fluidized bed: agglomeration by recrystallization, film-forming binders, and layering. Agglomeration by recrystallization is a solution for materials that are soluble in water. Generally, very hydrophilic fine powders, when placed in water wet rapidly on the surface, but tend to form a mucous around the bulk of the powder. Dispersibility is dramatically improved using this technique. Agglomeration by film-forming binders is utilized when materials that are insoluble in water are used and where granule strength is an issue. Granule size and strength are a function of the type and concentration of binder and, in general, the agglomerates have a much lower internal porosity than those produced by recrystallization. Agglomeration by layering is used to form small agglomerates; primary particles become attached to the nuclei and several agglomerates coalesce into larger ones. The difference between these granules and those produced by other techniques is that there is little internal porosity.

In the dry granulation method, dry powder particles may be brought together mechanically by compression into slugs or, more frequently, by roller compaction. Roll compaction includes compacting uniformly mixed powders between two counter-rotating roll pairs to form a compressed sheet that is then milled or granulated. Roll compaction processes have been found to be very effective at stabilizing mixtures of particles of different dimensions and properties which is advantageous for combining carrier compounds, sweetener molecules, flavors, and other materials.

It is to be noted that, in one particular embodiment, subtilisin is roll compacted with an encapsulant

(e.g., a starch hydrolysate), the resulting encapsulated enzyme having, for example, about a 45 wt% loading, about a 50 wt% loading or about a 55 wt% loading of the enzyme; that is, the substilisin constitutes about 45 wt%, about 50 wt% or about 55 wt% of the encapsulated enzyme. The encapsulated enzyme is well suited for use in, for example, a confectionary product.

It is to be noted that, in yet another particular embodiment, trypsin is agglomerated with a solid material (e.g., a carbohydrate, such as corn syrup solids), the resulting agglomerated enzyme having, for example, about a 10 wt% loading, about a 15 wt% loading, or about a 20 wt% loading of the enzyme; that is, the trypsin constitutes about 10 wt%, about 15 wt%, or about 20 wt% of the encapsulated enzyme. The encapsulated enzyme is well- suited for use in, for example, a pressed mint.

Encapsulated protease enzymes may also be prepared by absorbing the enzyme onto another component which is porous, to entrap the enzyme in the matrix of the porous component. Common materials used for absorbing the protease enzyme include, but are not limited to, silicas, silicates, polymers, pharmasorb clay, spongelike beads or microbeads, amorphous sugars like spray-dried dextrose, sucrose, alditols, amorphous carbonates and hydroxides, including aluminum and calcium lakes, vegetable gums and other spray dried materials. Depending on the type of absorbent material and how it is prepared, the amount of protease enzyme material that can be loaded onto the absorbent will vary. Typically, however, the enzyme loading may range from about 25 wt% to about 200 wt%, about 50 wt% to about 150 wt%, or about 75 wt% to about 125 wt% (based on the total weight of the support material). For example, materials like silicas and pharmasorb clays may be able to absorb about 50 wt% to about 150 wt% of the enzyme, based on the weight of the support material.

Typically, the procedure for absorbing the protease enzyme onto the absorbent support comprises mixing an absorbent, such as fumed silica powder in a powder blender, and spraying an aqueous solution of a slightly soluble protease enzyme material onto the powder while mixing continues. Generally, water is the solvent, but other solvents like alcohol could also be used if approved for use in food. As the powder mixes, the liquid is sprayed onto the powder. Spraying is stopped before the mix becomes damp. The still free-flowing powder is removed from the mixer, dried to remove the water or other solvent, and then ground to a specific particle size. After the protease enzyme is absorbed onto an absorbent support, or otherwise fixed onto an absorbent support, the protease enzyme may be encapsulated by one or more of the methods detailed herein, or as generally known in the art. Either full or partial encapsulation may be used, depending on the coating composition used in the process. Full encapsulation may be obtained, for example, by coating using spray drying, spray chilling, fluid-bed coating, coacervation or any other standard technique. A partial encapsulation or coating can be obtained by agglomeration of the fixative/protease enzyme mixture using any of the materials discussed above, or as generally known in the art.

The protease enzyme may be coated in a single step process, incorporating one of the methods detailed herein, or a multiple step process, incorporating more than one process detailed herein. For example, the protease enzyme may be encapsulated with any of the materials, as described previously, and then the encapsulated protease enzyme can be agglomerated, also as described previously, to obtain an encapsulated/agglomerated protease enzyme product that may be used in an oral composition.

### 2. Oral Compositions

Oral compositions in which coated or encapsulated protease enzymes may be incorporated, to provide a composition having an improved ability to remove stains from teeth surfaces (e.g., teeth whitening) include for example chewing gums (e.g., compressed gums), confections (e.g., hard and chewy candies), nougats, chocolates, toffees, dragees, caramels, lozenges, pressed tablets, capsules, edible films, dentrifices, nuts, foams, mouthwashes, mouthsprays, toothpaste products, and combinations thereof. Typically, the coated or encapsulated protease enzyme constitutes at least about 0.02 wt%, at least about 0.05 wt%, at least about 0.1 wt%, at least about 0.25 wt%, at least about 0.5 wt%, or at least about 1 wt% of the oral composition. Additionally, the encapsulated protease enzyme typically constitutes less than about 10 wt%, less than about 8 wt%, less than about 6 wt%, or less than about 4 wt% of the oral composition. The concentration of the coated or encapsulated enzyme in the oral composition may therefore range from about 0.02 wt% to about 10 wt%, or from about 0.1 wt% to about 8 wt%, or from about 0.5 wt% to about 6 wt%, or from about 1 wt% to about 4 wt%.

In this regard it is to be noted that the amount of encapsulated protease enzyme present in the oral composition may, at least in part, be a function of the enzyme loading in the encapsulated enzyme (e.g., higher loading requiring less to be added to the oral composition). Accordingly, in various alternative embodiments, the encapsulated protease enzyme may constitute from about 0.02 wt% to about 10 wt% of the oral composition, or from about 0.03 wt% to about 8 wt% of the oral composition, or from about 0.05 wt% to about 7 wt% of the oral composition. In various such embodiments, the encapsulated protease enzyme may constitute a portion of the oral composition at or near the lower ends of these ranges; for example, the encapsulated protease enzyme may constitute from about 0.02 wt% to about 1 wt%, from about 0.03 wt% to about 0.75 wt%, or from about 0.05 wt% to about 0.5 wt% of the oral composition. In still other alternative embodiments, the encapsulated protease enzyme may constitute from about 2 wt% to about 10 wt% of the oral composition, or from about 4 wt% to about 8 wt%, or from about 5 wt% to about 7 wt% of the oral composition. In various other embodiments (e.g., compressed and/or tabletted chewing gum compositions), the encapsulated protease enzyme may constitute from about 5 wt% to about 10 wt% of the oral composition, or from about 6 wt% to about 10 wt% of the oral composition.

Generally speaking, the oral composition of the present invention may be prepared by any means or method known to one of ordinary skill in the art. Typically, however, the oral composition is prepared by a method that comprises contacting an encapsulated protease enzyme with one or more components of the oral composition to form a mixture. For example, the method typically includes placing the encapsulated enzyme in a vessel containing the one or more components of the oral composition, or the finished oral composition but for the encapsulated enzyme (i.e., the encapsulated enzyme is added last). Generally, the method further comprises forming the mixture of components into a suitable shape for oral consumption.

In those embodiments in which a chewing gum is prepared, the one or more components may generally be selected from a sweetener, gum base, a flavor, a polyol, and combinations thereof. When a compressed chewing gum is desired, the one or more components of the oral composition generally include a powdered gum base. By way of further example, when the oral composition to be prepared is a pressed tablet, the one or more components contacted with the encapsulated protease enzyme generally comprise a polyol selected from the group consisting of mannitol, xylitol, sorbitol, maltitol, hydrogenated isomaltalose, lactitol, erythritol, and combinations thereof.

As noted elsewhere herein, encapsulation or coating of the protease enzyme surface may provide greater stability to the enzyme during preparation and/or storage of the oral composition. However, in some embodiments, the manner by, or conditions under, which the coated or encapsulated enzyme is incorporated into the oral composition (e.g., a compressed gum composition) may be optimized to further limit, if not avoid, substantial denaturation or deactivation of the enzyme. Among the conditions of the oral composition manufacture that may contribute to denaturation of the encapsulated enzyme are the process temperature, or more particularly the temperature at which the encapsulated enzyme is contacted with the other component(s) of the oral composition (e.g., the temperature of the mixture containing the encapsulated enzyme and one or more other components of the composition) and/or the pH of the other component(s) of the oral composition, with which the encapsulated enzyme is contacted or mixed. Enzyme denaturation or deactivation is, in general, directly proportional to the temperature, and/or pH, of the other component or components with which the encapsulated enzyme is contacted or mixed.

Therefore, in accordance with the present invention, the temperature at which the encapsulated protease enzyme is added to the mixture of components of the oral composition generally does not substantially denature the enzyme. For example, this temperature is typically less than about 70°C, less than about 60°C, or even less than about 50°C. Furthermore, the pH of the component, or mixture of components, to which the encapsulated protease enzyme is added or mixed and/or the finished composition generally does not substantially denature the enzyme and may be at least about 2, at least about 4, or at least about 6, the pH for example being greater than about 2 and less than about 8, greater than about 5 and less than about 7.5, or greater than about 6 and less than about 7. Additionally or alternatively, the mixture to which the encapsulated protease enzyme is added generally has a moisture content that does not substantially denature the enzyme.

### A. Chewing Gums

In various embodiments of the present invention, the oral composition is in the form of a chewing gum. The chewing gum of the present invention can be prepared using a variety of different methods and machinery known in the art. For example, the formulation can be prepared in individual batches, using for example a sigma blade mixer. Alternatively, however, the formulation may be prepared by continuous processing, using equipment known in the art. Conventional sheeting and scoring machinery can be used to form and score the chewing gum centers, or the centers can be made on a forming machine that involves a drop frame and nitrogen cooling allowing spheres, ovals, and other shapes to be prepared. A variety of different tableting processes may also be used, provided they allow for preparation of a uniform gum center. Standard tableting techniques include, but are not limited to, direct compression and granulation (wet or dry methods). Conventional drug tableting equipment or confectionary tableting product equipment may also be utilized. An example of such equipment is the Stokes tableting machine, available from Stokes Manufacturing Inc.

A chewing gum composition generally comprises a water-soluble bulk portion, a water-insoluble chewable gum base portion, and one or more water-soluble flavoring agents. The water-soluble bulk portion dissipates with a portion of the flavoring agent(s) over a period of time during chewing while the gum base portion is retained in the mouth throughout the chew. The amount of gum base used in a chewing gum composition may depend on a number of factors, including for example the desired chew characteristics and/or other physical properties of the gum.

The insoluble gum base generally comprises elastomers, resins, fats and oils, softeners, and inorganic fillers and may also include wax. The insoluble gum base may constitute about 5 wt% to about 95 wt% of the chewing gum composition. However, the gum base typically constitutes from about 10 wt% to about 50 wt% of the chewing gum and, more typically, from about 25 wt% to about 35 wt% of the chewing gum.

In various embodiments, the chewing gum base may contain from about 20 wt% to about 60 wt% of a synthetic elastomer, up to about 30 wt% of a natural elastomer, from about 5 wt% to about 55 wt% of an elastomer plasticizer, from about 4 wt% to about 35 wt% of a filler, from about 5 wt% to about 35 wt% of a softener, and optional minor amounts (e.g., about 1 wt% or less) of miscellaneous ingredients or components such as colorants, antioxidants, etc.

Synthetic elastomers may include, but are not limited to, polyisobutylene having a GPC weight average molecular weight of about 10,000 to about 95,000, isobutylene-isoprene copolymer (butyl elastomer), styrene-butadiene copolymers (having styrene-butadiene ratios of, for example, about 1:3 to about 3:1), polyvinyl acetate having GPC weight average molecular weight of about 2,000 to about 90,000, polyisoprene, polyethylene, vinyl acetate-vinyl laurate copolymer having vinyl laurate content of about 5 wt% to about 50 wt% of the copolymer, and combinations thereof.

Preferred synthetic elastomers include polyisobutylene having a GPC weight average molecular weight of from about 50,000 to 80,000, styrene-butadiene copolymers having a styrene-butadiene ratio for bound styrene of from 1:1 to 1:3, polyvinyl acetate having a GPC weight average molecular weight of from 10,000 to 65,000, with the higher molecular weight polyvinyl acetates typically used in bubble gum base, and vinyl acetate-vinyl laurate copolymer having a vinyl laurate content of 10.

Natural elastomers may include natural rubber, such as smoked or liquid latex and guayule, as well as natural gums, such as jelutong, lechi caspi, perillo, sorva, massaranduba balata, massaranduba chocolate, nispero, rosindinha, chicle, gutta hang kang, and combinations thereof. The preferred synthetic elastomer and natural elastomer concentrations vary depending on whether the chewing gum in which the base is used is adhesive or conventional, bubble gum or regular gum. Preferred natural elastomers include jelutong, chicle, sorva, and massaranduba balata.

Elastomer plasticizers may include, but are not limited to, natural rosin esters such as glycerol esters or partially hydrogenated rosin, glycerol esters of polymerized rosin, glycerol esters of partially dimerized rosin, glycerol esters of rosin, pentaerythritol esters of partially hydrogenated rosin, methyl and partially hydrogenated methyl esters of rosin, pentaerythritol esters of rosin; synthetics such as terpene resins derived from alpha, beta, and/or any suitable combinations of the foregoing. The preferred elastomer plasticizers will also vary depending on the specific application, and on the type of elastomer which is used.

Fillers and/or texturizers may include magnesium and calcium carbonate, ground limestone, silicate types such as magnesium and aluminum silicate, clay, alumina, talc, titanium oxide, mono-, di- and tri-phosphate, cellulose polymers, such as wood, and combinations thereof.

Softeners and/or emulsifiers may include tallow, hydrogenated tallow, hydrogenated and partially hydrogenated vegetable oils, cocoa butter, glycerol monostearate, glycerol triacetate, lecithin, mono and triglycerides, acetylated monoglycerides, fatty acids (e.g. stearic, palmitic, oleic and linoleic acids), and combinations thereof.

Colorants and whiteners may include FD&C dyes and lakes, fruit and vegetable extracts, titanium dioxide, and combinations thereof.

As noted elsewhere herein, the gum base may include wax. However, an example of a wax-free gum base is disclosed in U.S. Patent No. 5,286,500.

In addition to a water insoluble gum base portion, a typical chewing gum composition further includes a water soluble bulk portion. The water soluble portion can include bulk sweeteners, high intensity sweeteners, flavoring agents, softeners, emulsifiers, colors, acidulants, fillers, antioxidants, and other components that provide desired attributes.

Softeners may be added to the chewing gum in order to optimize the chewability and mouthfeel of the gum. The softeners, which are also known as plasticizers and plasticizing agents, generally constitute from about 0.5 wt% to about 15 wt% of the chewing gum. The softeners may include glycerin, lecithin, and combinations thereof. Aqueous sweetener solutions such as those containing sorbitol, hydrogenated starch hydrolysates, corn syrup, and combinations thereof, may also be used as softeners and binding agents in the chewing gum.

Bulk sweeteners, or bulking agents, include both sugar and sugarless components. Bulk sweeteners typically constitute from about 5 wt% to about 95 wt% of the chewing gum, more typically from about 20 wt% to about 80 wt% of the chewing gum and, more typically, from about 30 wt% to about 60 wt% of the gum. Sugar sweeteners generally include saccharide components commonly known in the chewing gum art, including but not limited to, sucrose, dextrose, maltose, dextrin, dried invert sugar, fructose, levulose, galactose, corn syrup solids, and the like, alone or in combination. Sugarless sweeteners include, but are not limited to, sugar alcohols such as sorbitol, mannitol, xylitol, maltitol, hydrogenated starch hydrolysates, erythritol, tagatose, trehalose, and the like, alone or in combination.

High intensity artificial sweeteners can also be used, alone or in combination, with the above. Preferred sweeteners include, but are not limited to, sucralose, aspartame, NAPM derivatives such as neotame, salts of acesulfame, alitame, saccharin and its salts, cyclamic acid and its salts, glycyrrhizinate, dihydrochalcones, thaumatin, monellin, and the like, alone or in combination. In order to provide longer lasting sweetness and flavor perception, it may be desirable to encapsulate or otherwise control the release of at least a portion of the artificial sweetener. Such techniques as wet granulation, wax granulation, spray drying, spray chilling, freeze-drying, fluid bed coating, coacervation, and fiber extension may be used to achieve the desired release characteristics.

In accordance with the present invention, it has been discovered that high intensity sweeteners having a peptide structure, such as aspartame, neotame, or other salt derivatives such as acesulfame-k, may not preferred in certain embodiments unless they are encapsulated. If such a sweetener is placed neat in an oral composition of the present invention, it is typically broken down within 1 to 2 weeks, resulting in production of sulfide by-products that create sulfur sensory off-notes when chewed. Benchtop sensory tests of compressed chewing gum made with and without acesulfame-k have shown that sulfur notes were not generated with the gum that did not contain acesulfame-k.

Combinations of sugar and/or sugarless sweeteners may be used in the chewing gum. Additionally, the softener may also provide additional sweetness such as with aqueous sugar or alditol solutions.

If a low calorie gum is desired, a low calorie bulking agent can be used. Examples of low calorie bulking agents include polydextrose, raftilose, raftilin, fructooligosaccharides (e.g., NutraFlora®), Palatinose oligosaccharide, guar gum hydrolysate (e,g., Sun Fiber®), or indigestible dextrin (e.g., Fibersol®).

Flavoring agents that provide the desired flavors may include essential oils, synthetic flavors, or mixtures thereof, including, but not limited to, oils derived from plants and fruits such as citrus oils, fruit essences, peppermint oil, spearmint oil, other mint oils, clove oil, oil of wintergreen, anise and the like. Artificial flavoring agents and components may also be used. Flavoring agent(s) may be present in powder or liquid form and natural and artificial flavoring agents may be combined in any sensorial acceptable fashion. Flavoring agent(s) may include a cooling agent to enhance the flavor and perceived breath freshening of the product. Cooling agents include menthol, ethyl p-menthane carboxamide, N,2,3-trimethyl-2-isopropyl-butanamide, menthyl glutarate (Flavor Extract Manufacturing Association (FEMA 4006)), menthyl succinate, menthol PG carbonate, menthol EG carbonate, menthyl lactate, menthone glyceryl ketal, menthol glyceryl ether, N-tertbutyl-p-menthane-3-carboxamide, p-menthane-3-carboxylic acid glycerol ester, methyl-2-isopryl-bicyclo (2.2.1), heptane-2-carboxamide, menthol methyl ether and combinations thereof. Any of these flavoring agents may be present in amounts of from about 0.1 wt% to about 15 wt% of the chewing gum and, more typically, in amounts from about 0.2 wt% to about 5 wt% of the chewing gum.

Optionally, the chewing gum of the present invention may include additional breath freshening, anti-microbial or oral health ingredients or components, such as food acceptable metallic salts selected from zinc and copper salts of gluconic acid, zinc and copper salts of lactic acid, zinc and copper salts of acetic acid, zinc and copper salts of citric acid, copper chlorophyll and combinations thereof. The chewing gum may also include anti-microbial essential oils and flavor components such as peppermint, methyl salicylate, thymol, eucalyptol, cinnamic aldehyde, polyphosphate, pyrophosphate and combinations thereof may be added to the gum composition. Dental health ingredients or components, such as fluoride salts, phosphate salts, lipids, anti-microbials, calcium, electrolytes, protein additives, dental abrasives and combinations thereof may also be added to the gum composition.

In addition to the active ingredients of the present invention, additional active ingredients or medicaments may be added for various purposes. If the medicament or active is water soluble in the chewing gum, it preferably will include a base/emulsifier system which leads to the desired concentration of the medicament in the saliva (i.e., a more hydrophilic balance). If the medicament or active is water insoluble, the chewing gum preferably includes a base/emulsifier system which leads to the desired concentration of the medicament in the saliva (i.e., a more lipophilic balance).

In general, the chewing gum is manufactured by sequentially adding the various chewing gum ingredients or components to a commercially available mixer known in the art. The encapsulated protease enzyme may generally be added at any time during the manufacturing process but, preferably, is added near the end of mixing in order to minimize exposure of the enzyme to heat that could possible denature the enzyme. After the ingredients or components have been thoroughly mixed, the gum mass is discharged from the mixer and shaped into the desired form such as rolling sheets and cutting into sticks, extruding into chunks or casting into pellets, which are then coated or panned.

Generally, the ingredients or components are mixed by first melting the gum base and adding it to the running mixer; the base may also be melted in the mixer itself. Color or emulsifiers may also be added at this time. A softener such as glycerin may also be added at this time, along with syrup and a portion of the bulking agent. Further parts of the bulking agent are added to the mixer. Flavoring agents are typically added with the final portion of the bulking agent. Other optional ingredients or components are added to the batch in a typical fashion, well known to those of ordinary skill in the art.

The entire mixing procedure typically takes from five to fifteen minutes, but longer mixing times may sometimes be required. Those skilled in the art will recognize that many variations of the above described procedure may be followed.

Chewing gum base and chewing gum product have been manufactured conventionally using separate mixers, different mixing technologies and, often, at different factories. One reason for this is that the optimum conditions for manufacturing gum base, and for manufacturing chewing gum from gum base and other ingredients or components such as sweeteners and flavors, are so different that it has been impractical to integrate both tasks. Chewing gum base manufacture, on the one hand, involves the dispersive (often high shear) mixing of difficult-to-blend ingredients or components such as elastomer, filler, elastomer plasticizer, base softeners/emulsifiers and sometimes wax, and typically requires long mixing times. Chewing gum product manufacture, on the other hand, involves combining the gum base with more delicate ingredients or components such as product softeners, bulk sweeteners, high intensity sweeteners and flavoring agents using distributive (generally lower shear) mixing, for shorter periods.

It is to be noted that, in one particular embodiment of the present invention, the encapsulated enzyme of the present invention may be incorporated into a compressed chewing gum composition, using processing means and gum formulations otherwise known in the art (see, for example, U.S. Patent Nos. 2,290,120, 4,753,805, 5,582,852, 6,290,985, 6,322,828, 6,582,738, and 6,558,722; U.S. Patent Publications Nos. 2003/0086999, 2003/0026871, 2003/0099741, and WO 2003/084338).

In accordance with such embodiments, typically the moisture content (e.g., liquid flavor content) of the component, or mixture of components, to which the encapsulated enzyme is added or mixed, the resulting mixture and/or finished composition is less than about 6 wt%, less than about 4 wt%, less than about 2 wt%, or even less than about 1 wt%.

Generally, any standard technique for compressing a granular composition into a particular shape may be employed to prepare the granulated chewing gum composition of the present invention and form a compressed shape (e.g., a tablet) thereof. For example, one common technique for compressing a granular composition is tableting. Generally, tableting involves the use of a tablet press, which comprises a die and a punch. The basic principles of compression apply, wherein the die is filled with the granular chewing gum composition which is then compressed by the punch being lowered under pressure. This pressure on the composition is maintained for a period of time, known as the dwell time, which is sufficient to bond the granular particles of the composition together and compact them to form the compressed chewing gum shape (e.g., tablet). Once formed, the tablet is ejected from the die.

A process for preparing the tableted gum center may generally proceed by adding shredded gum base to an unheated gum mixer, preferably a double sigma blade. Along with the gum base is added powdered color and one-third of the portion of sugar or, sweetener, that will be used. Mixing is begun and after the sugar incorporates into the gum base, which typically takes from approximately 5 to 7 minutes, the second one-third portion of the sugar or sweetener is added to the mixer. Once the mixture appears homogenous the remaining sugar or sweetener portion (i.e., the final third) is added to the mixer. The total mixing time is typically approximately 25 minutes. The resulting powder is removed from the mixer and sifted to a desired mesh size. An appropriate amount of spray dried flavor, acid, tableting agents (e.g., magnesium stearate) and flow agents (e.g., silicone dioxide) may then be added. This final mixture is then mixed until the powder is homogenous. The resulting powder is then pressed on a tableting machine such as, for example, a Stokes machine.

Another common technique for compressing a granular composition is briquetting. This technique is described in, for example, International Application No. WO 99/25203.

Regardless of the precise manner selected for preparing the compressed chewing gum, processing times, as well as processing techniques, may depend on a number of factors including, for example, the components of the gum base, the desired properties of the end product gum base, and, in the case of a batch process, the size of the batch being prepared. For example, compounding of the mixer contents typically begins to be effective once the ingredients have become homogenous. Accordingly, compounding time generally varies, typically ranging from about 15 minutes to about 90 minutes.

In various embodiments, a continuous process, using, for example, a mixing extruder, may be used to prepare the gum base. For example, after the initial ingredients have been combined to form a homogeneous mixture and been compounded for the time desired, the remaining base ingredients may be added, concurrently or sequentially, to form a homogeneous molten mass. For example, any remainder of the elastomer and/or the plasticizer may be added after the initial compounding time. Other optional components, such as waxes and/or oils, may be added in this manner and/or after the elastomer and plasticizer have been introduced. The mass is then allowed to become homogeneous before discharging.

Generally speaking, and as noted elsewhere herein, the insoluble gum base portion of the granular chewing gum composition of the present invention may be essentially any gum base that possesses the desired properties, and that can be uniformly distributed as small particles throughout the granular chewing gum composition when the gum base is mixed with the other components thereof in a mixing apparatus, as described elsewhere herein.

The water-soluble bulk portion of granulated (i.e., compressed) chewing gum compositions of the present invention may additionally include, for example, one or more of the following: flavoring agents, bulk sweeteners or high intensity sweeteners, granulating agents, softeners, emulsifiers, coloring agents, acidulants, fillers, antioxidants, and/or lubricants, as well as other components that are known in the art. Generally, the precise combination of components, as well as the concentrations thereof, may be determined by means known in the art, in order to obtain a chewing gum composition having the desired properties.

The type(s), as well as amount(s), of the flavoring agent(s) added to the granular chewing gum composition may vary due to a number of considerations including, for example, the desired strength and/or duration of the flavor the composition is to possess. Additionally, the amount of flavoring agent added to the granular chewing gum composition may also vary with the type of flavoring agent to be added, as some agents are more intense than others. In at least one embodiment, the concentration of flavoring agent is at least about 0.5 wt% of the granular chewing gum composition, at least about 1 wt%, at least about 2.5 wt%, at least about 5 wt% or more (e.g., at least about 6 wt%, at least about 10 wt% or more), the concentration for example falling within the range of from about 1 wt% to about 10 wt%, from about 2 wt% to about 8 wt%, or from about 4 wt% to about 6 wt% of the granular chewing gum composition. It is to be noted that, in accordance with various embodiments of the present invention, it is advantageous for the temperature throughout the oral composition preparation process to be kept relatively low, as noted elsewhere herein. Without being held to any particular theory, it is believed that processing at such relatively low temperatures is advantageous by, in addition to limiting or preventing denaturing of the encapsulated enzyme, limiting or preventing degradation of thermally unstable flavoring agents which may be prone to degradation at higher temperatures. Flavoring agents which are useful in a chewing gum produced by the present process include, for example, natural and artificial or synthetic flavorings, or a combination thereof, including essential oils, essences, extracts, powders, including acids and other substances capable of affecting the taste profile. Although the range of flavors suitable for use in compressed chewing gums is nearly limitless, they commonly fall into several broad categories.

Fruit flavors include lemon, orange, lime, grapefruit, tangerine, strawberry, apple, cherry, raspberry, blackberry, blueberry, banana, pineapple, cantaloupe, muskmelon, watermelon, grape, currant, mango, kiwi and many others as well as combinations thereof. Mint flavors include spearmint, peppermint, wintergreen, basil, corn mint, menthol and mixtures thereof. Spice flavors include cinnamon, vanilla, clove, chocolate, nutmeg and many others. Less commonly used are herbal and savory flavors, such as popcorn, chili, corn chip and the like.

The flavoring agent may also include a cooling agent to enhance the flavor and perceived breath freshening of the product. In addition to menthol, cooling agents may include, for example, ethyl p-menthane carboxamide, N-2,3-trimethyl-2-isopropylbutanamide, menthyl glutarate, menthyl succinate, menthol PC carbonate, menthol EC carbonate, menthyl lactate, menthone glyceryl ketal, menthol glyceryl ether, N-tertbutyl-p-menthane-3-carboxamide, p-menthane-3-carboxylic acid glycerol ester, methyl-2-isopryl-bicycle (2.2.1), heptane-2-carboxamide, menthol methyl ether and combinations thereof. The chewing gum of the present invention may also optionally include other breath freshening or anti-microbial ingredients, including anti-microbial essential oils and flavor components, such as peppermint, methyl salicylate, thymol, eucalyptol, cinnamic aldehyde, polyphosphate, pyrophosphate and combinations thereof, may also be used.

It is to be noted that the flavoring agent may be added as a liquid, a solid or both. For example, in some embodiments, a compressed chewing gum composition of the present invention may contain a spray dried flavor as a partial or complete replacement of a liquid flavor. Alternatively, the flavorant may be in the form of a freeze-dried solid (e.g., a powder). When a combination of liquid and solid flavoring agents is used, the weight ratio of the liquid flavorant to the solid flavorant may range, for example, from between about 5:1 to about 1:5, or about 2:1 to about 1:2. In at least one embodiment, the liquid flavorant is slightly in excess, the weight ratio of liquid flavorant to solid flavorant being, for example, between about 1:1 and about 1.5:1 (e.g., about 1.2:1 or about 1.3:1).

Similar to the flavoring agent, the type(s), as well as amount(s), of coloring agent(s) added to the granular chewing gum composition may vary due to a number of considerations including, for example, the desired intensity and/or duration of the color the composition is to possess, the precise concentration used being determined using means standard in the art. Additionally, the amount of coloring agent added to the granular chewing gum composition may also vary with the type of coloring agent to be added, as some agents are more intense than others. Exemplary coloring agents include, for example, commonly used colors or whiteners (e.g., FD&C dyes and lakes, fruit and vegetable extracts, titanium dioxide, etc.).

A processing aid, such as a lubricating agent, may also optionally be included in the granular chewing gum composition, the processing aid, such as a lubricant, being one or more substances that help to keep the granular chewing gum composition in free-flowing particulate form, both during the formation of the chewing gum formulation and afterwards. Furthermore, the presence of the processing aid may also result in a granular chewing gum composition that is less sticky, which reduces the amount of the composition that sticks to the interior surfaces and blades of the mixing equipment, and therefore remains inside the mixing equipment after each production run. By reducing the amount of the chewing gum composition that remains inside the mixing equipment after each production run, the yield of chewing gum composition from each production run may be increased (e.g., yields of, for example, at least about 80%, at least about 90%, at least 95%, or more, of the chewing gum composition being obtained). Further, the mixing equipment, or other processing equipment, may be cleaned more easily and thoroughly, thereby resulting in less down time between production runs.

Like the flavoring agents and the coloring agents, the type(s), as well as amount(s), of the processing aid (e.g., lubricating agent), added to the granular chewing gum composition may vary due to a number of considerations including, for example, whether the granular chewing gum composition is to be compressed into a tablet, the processing aid, for example, aiding in the tabletting of the granular composition. Additionally, the amount of processing aid added to the granular chewing gum composition, for example prior to tabletting, may also vary with the type of aid to be added, as some are more effective than others. In one embodiment, the concentration of the processing aid, such as a lubricant, is at least about 0.5 wt% of the granular composition, at least about 1 wt%, at least about 2 wt%, at least about 3 wt%, at least about 4 wt%, at least about 5 wt% or more (e.g., at least about 6 wt%, at least about 8 wt%, or more), the concentration for example falling within the range of from about 0.5 wt% to about 5 wt%, or from about 1 wt% to about 4 wt%. Exemplary processing aids include lubricants such as, for example, stearates (e.g., magnesium stearate), sodium stearyl fumarate, hydrogenated vegetable oils, talc, silica (e.g., fumed silica or precipitated silica), or stearic acid, with stearates being used in one particular embodiment.

Additionally, the granular chewing gum composition may optionally contain a granulating agent, which in general is a substance that does not adversely react with the other components of the granular chewing gum composition and results in a chewing gum composition with the desired properties when mixed with the other components, according to the process of the present invention. For example, the granulating agent may be, in at least some embodiments, water-soluble, so that the final granular chewing gum composition will have better organoleptic properties. In one particular embodiment, the granulating agent also functions as a sweetening agent.

Compressed gums of the present invention may also include one or more sweetening agents (e.g., one or more bulk sweeteners). Suitable sweetening agents are generally known in the art and, when used in the method of the present invention, generally aid in forming a compressed chewing gum composition with the desired properties. Examples of potential sweeteners for use in the granular chewing gum composition of the present invention include carbohydrates, particularly sugars (e.g., such as sucrose, dextrose, maltose, dextrin, glucose, fructose, levulose, galactose, dried invert sugars, corn syrup solids, and the like, alone or in combination), and/or sugarless sweeteners, such as tagatose, trehalose, sugar alcohols or polyols (e.g., sorbitol, mannitol, maltitol, xylitol, isomalt and erythritol, and/or a combination thereof), and/or high intensity artificial sweeteners (which are sometimes referred to as high potency or artificial sweeteners, and which may be defined as food acceptable chemicals which are at least about 10 or about 20 times sweeter than sucrose), such as glycine, aspartame, sucralose, NAPM derivates such as neotame, salts of acesulfame (e.g., acesulfame K), alitame, saccharin and its salts, cyclamic acid and its salts, cyclohexyl sulfamate, stevioside and glycyrrhizinate (e.g., ammonium glycyrrhizinate), dihydrochalcones, thaumatin, monellin, perilla-derived sweeteners, stevia-derived sweeteners, monatin, monellin, chalcones, as well as sweetener-sweetener salt combinations (e.g.,aspartame-acesulfame salt), and the like, as well as mixtures thereof.

In at least one embodiment, the sweetening agent, or bulk sweetener, is added as a dry particulate or powder.

It is to be noted that certain substances can function as both a sweetening agent and a granulating agent, such as for example sugars (like sucrose, fructose, dextrose and mixtures thereof) and polyols (like sorbitol, mannitol, isomalt, xylitol, erythritol, and mixture thereof). When one or more of these substances is used in the chewing gum composition as a granulating agent, there may be no need for a separate or different sweetening agent.

As with the other components (e.g., flavoring agents, coloring agents, etc.) added to the granular chewing gum composition, the type(s), as well as amount(s), of the sweetening agent(s) added to the granular chewing gum composition may vary due to a number of considerations, including for example the desired intensity and/or duration of the sweetness the composition is to possess. Additionally, the amount of sweetening agent added to the granular chewing gum composition may also vary with the type of sweetening agent to be added, as some agents are more intense than others. In at least one embodiment, the concentration of sweetening agent is at least about 25 wt% of the granular composition, at least about 35 wt%, at least about 45 wt%, at least about at least 55 wt%, at least about 65 wt%, at least about 75 wt%, or more, the concentration for example falling within the range of from about 25 wt% to about 75 wt%, from about 35 wt% to about 65 wt%, or from about 45 wt% to about 55 wt% of the granular composition. Additionally, it is to be noted that, when an intense sweetener is used in the granular chewing gum composition, the concentration thereof may, for example, typically fall within the range of from about 0.01 wt% to about 2 wt%, from about 0.1 wt% to about 1.5 wt%, or from about 0.5 wt% to about 1 wt% of the granular chewing gum composition.

A softener may also optionally be added to a compressed chewing gum of the present invention, in order to optimize chewability and/or "mouthfeel" of the gum. A softener, which may also be known as a plasticizer or plasticizing agent, may, for example, constitute between about 0.5 wt% to about 15 wt% of the chewing gum, or from about 1 wt% to about 10 wt% of the chewing gum, or from about 2 wt% to about 8 wt% of the chewing gum. The softeners may include, for example, glycerin, lecithin, triacetin (or glyceroltriacetate), and combinations thereof.

As previously noted, emulsifiers may also be used to modify the texture and cause the hydrophobic and hydrophilic components of the chewing gum composition to be miscible. Emulsifiers suitable for use in the present invention may include, for example, glycerol monostearate, glycerol triacetate, lecithin, mono- or didiglycerides, acetylated mono- or di- glycerides, and distilled mono- or diglycerides.

It is to be noted that many shapes and sizes of a tablet may be made by varying the shape of the die and punch (e.g. circular, briquette, pillow, etc.).

Examples of chewing gum formulations, including compressed chewing gums, containing one or more encapsulated enzymes are set forth below in Examples 25-34.

### B. Tablets and Lozenges

In various other embodiments, the oral composition is in the form of a pressed tablet (e.g., pressed mint) or lozenge (e.g., a boiled drop) manufactured using conventional techniques. Preferably, in various embodiments, these tablets or lozenges are slow dissolving. Generally, pressed tablets and lozenges are prepared in the same manner with the same general formula; however, lozenge formulations typically contain up to about 2 wt% of a hydrocolloid as a barrier agent to provide a shiny surface while pressed tablets do not contain a barrier agent and typically have a smooth finish. Typical tablet formulations are set forth below in Examples 35-39.

Generally, a tablet is a mixture of base materials, binders, flavors, and lubricants. The base material is typically a sugar or a polyol. Pressed tablets and lozenges of the present invention typically comprise a solid carrier in the form of a sugar or a water soluble polyhydric alcohol (polyol) such as mannitol, xylitol, sorbitol, maltitol, hydrogenated isomaltalose, lactitol, erythritol, a hydrogenated starch hydrolysate (e.g., "Lycasin"), hydrogenated glucose, hydrogenated disaccharides, and/or hydrogenated polysaccharides, as the major ingredient or component, in an amount of from about 85 wt% to about 98 wt% of the carrier. Among the sugars that may be used are sucrose, dextrose, lactose, maltose, and other common sugars. Solid salts such as sodium bicarbonate, sodium chloride, potassium bicarbonate or potassium chloride may totally or partially replace the polyol carrier. High-intensity sweeteners such as acesulfame K, aspartame, alitame, sucralose, glycyrrhizin, saccharin and cyclamates may also be included with the base materials.

Binders that are commonly used are natural gums and hydrocolloids such as gum arabic, guar gum, agar, alginates, gum tragacanth, gelatin, corn syrup, starches and maltodextrins. Most commonly used binders are gelatin, gum arabic and maltodextrins or corn syrups. When non-sugar polyols such as sorbitol are used as the base material, binders are not needed for binding since many of these polyols are easily compressed to form tablets. In some cases polyols such as sorbitol may also act as a binder and may be combined with sugar to form the base materials for the compressed tablet. Binders usually comprise about 2 wt% to about 8 wt% of the tablet.

Lubricants are used to give good release from the press tooling or die and punches. A variety of lubricants or non-stick agents may be used in a tablet to act as release agents including, for example, acetylated monoglycerides, waxes, lecithins, emulsifiers, and mono-, di-, or tristearates. In particular, suitable lubricants may include vegetable oils (e.g., coconut oil), calcium stearate, magnesium stearate, amino acids, aluminum stearate, talc, starch, and Carbowax. The most common of these lubricants are magnesium or calcium stearate and stearic acid. Solid lubricants are added to the tablet composition to help form the tablet and allow for its release from the tablet press. Lubricants usually comprise about 0.1 wt% to about 5 wt% or from about 0.5 wt% to about 2 wt% of the tablet. In some instances, low levels of flow agents such as silicon dioxide are added to the tablet composition to help the flow of the mixture into the tablet press.

The tablets and lozenges generally include one or more flavorants. Suitable flavorants include natural and artificial flavors and mints, such as oil of peppermint, menthol, oil of spearmint, vanilla, oil of cinnamon, oil of wintergreen (methyl salicylate), anise, clove oil, lemon oil, orange oil, grape flavor, lime oil, grapefruit oil, apple, apricot essence, and combinations thereof. The flavorings are generally utilized in amounts that will vary depending upon the individual flavor, and may, for example, range in amounts of about 0.5 wt% to about 3 wt% or from about 0.01 wt% to about 2 wt% of the tablet or lozenge. Artificial flavoring components are also contemplated for use in tablets of the present invention. Those skilled in the art will recognize that natural and artificial flavoring agents may be combined in any sensorially acceptable blend. All such flavors and flavor blends are contemplated by the present invention.

In addition, menthol and physiological cooling agents (sometimes referred to as high-intensity coolants) may be added to the tablet at a level of about 0.01 wt% to about 1 wt%. Except for menthol, these cooling agents are preferably preblended with the flavor before being added to the mixture of ingredients or components used to form the tablet. Menthol may be preblended with the flavor or may be added to the tablet composition mixture in its crystalline form. Typical cooling agents include substituted p-menthane carboxamides, acyclic carboxamides, menthone glycerol ketals, menthyl lactate, menthyl succinate, and 3-1-menthoxypropane-1,2-diol. These cooling agents and flavors may also be incorporated into various coatings of the tablets.

Tablets and lozenges of the present embodiment typically also include optional sweeteners and or colorants.

Sweeteners may be one or more sweeteners known in the art, including both natural and artificial sweeteners. The sweetener may be chosen from a wide range of materials, including water-soluble sweeteners, water-soluble artificial sweeteners, and dipeptide based sweeteners and mixtures thereof. Thus, sweeteners may be chosen from the following non-limiting list, which includes sugars such as sucrose, glucose, corn syrup, dextrose, invert sugar, fructose, and mixtures thereof; saccharine and its various salts such as the sodium or calcium salt; cyclamic acid and its various salts such as the sodium salt; free aspartame; dihydrochalcone sweetening compounds; glycyrrhizin; stevioside; monellin, thaumatin, sucralose, isomaltitol, neotame, lactitol, trehalose, lactosucrose, polydextrose, tagatose, perillartine; and sugar alcohols such as sorbitol, sorbitol syrup, mannitol, maltitol, erythritol, xylitol, and the like. Also contemplated as a sweetener is the nonfermentable sugar substitute hydrogenated starch hydrolysate (also known as Lycasin). Also contemplated is the synthetic sweetener 3,6-dihydro-6-methyl-1-1,2,3-oxathiazin-4-one-2,2-dioxide, particularly the potassium (Acesulfame-K), sodium and calcium salts thereof. In various preferred embodiments, sorbitol is the sweetening and bulking agent. The amount of sweetener included is an amount effective to provide the desired degree of sweetness and bulk, generally from about 0.001 wt% to about 70 wt% by weight of the tablet or lozenge.

High intensity artificial sweeteners can also be used, alone or in combination, with other sweeteners. Preferred high intensity sweeteners include, but are not limited to, sucralose, aspartame, NAPM derivatives such as neotame, salts of acesulfame, alitame, stevia, saccharin and its salts, cyclamic acid and its salts, glycyrrhizinate, dihydrochalcones, thaumatin, monellin, and the like, alone or in combination. In order to provide longer lasting sweetness and flavor perception, it may be desirable to encapsulate or otherwise control the release of at least a portion of the artificial sweetener. Such techniques as wet granulation, wax granulation, spray drying, spray chilling, fluid bed coating, coacervation, and fiber extension may be used to achieve the desired release characteristics.

Colorants can be present in the tablets or lozenges of the present invention. Examples include pigments such as titanium dioxide, natural food colorants such as beta carotenes, betanin, turmeric, and other dyes suitable for food, drug and cosmetic applications known as F.D. & C. dyes, and the like. The materials may be incorporated in amounts of up to about 1 wt%, preferably up to about 6 wt% of the tablet or lozenge.

Tablets and lozenges of the present invention are generally prepared in accordance with conventional mixing and tableting techniques known in the art. For example, the pressed tablet may be prepared by wet granulation, dry granulation, and direct compression methods. In general, wet granulation involves mixing milled powders, preparing a wet mass by blending the milled powders with a binder solution, coarse screening the wet mass and drying the moist granules, screening the granules through a 14 to 20 mesh screen, mixing the screened granules with lubricants and disintegrate agents and finally tablet compressing the mass. In contrast, dry granulation generally involves milling of powders, compression into large hard tablets to make slugs, screening of slugs, mixing with lubricants and disintegrating agents and finally tablet compression. In the direct compression method, the milled ingredients or components are mixed and then merely tableted by compression. In the direct compression method, the base materials are dry blended along with any high-intensity sweeteners before any flavor is added. Liquid flavors and solid flavors are added slowly to the base materials and mixed in a dry material mixer, such as a ribbon mixer or a Hobart mixer. Lastly, the lubricant such as magnesium stearate is added, but not over mixed. Over mixing the mixture with magnesium stearate can reduce lubrication. In general, the final powder mixture is allowed to sit for up to 12 hours before being sent to the tablet press so that its properties will be suitable for tableting. If the mixture is too damp it may be dried before tableting.

Conventional rotary tablet presses are used to produce the preferred tablet including, for example, presses available from Fette America, 300 Roundhill Dr., Rockaway, N.J.; Stokes Div. of DT Industries, 1500 Grundy's Lane, Bristol, PA; or Thomas Engineering, Inc. 575 W. Central Rd., Hoffman Estates, IL. The basic steps of rotary tablet press operation include four steps. The first step is to fill the die cavity; the second step is to adjust the fill by removing excess fill; the third step is compression; and the fourth step is ejection of the tablet from the die. In standard production equipment, there is also a precompression step before the final compression and then ejection. Preferred confectionery tablets are about 0.2 to about 0.5 grams in size.

### C. Coatings

It is to be noted that, in various embodiments of the present invention, the oral composition (e.g., chewing gums and/or tablets) of the present invention may be coated. Conventional panning procedures may be used to coat the gum or tablet with sucrose, but recent advances in panning have allowed the use of other carbohydrate materials to be used in the place of sucrose. Some of these components include, but are not limited to, dextrose, maltose, palatinose, xylitol, lactitol, sorbitol, maltitol, mannitol, erythritol, hydrogenated isomaltulose and other new alditols or a combination thereof. These materials may be blended with panning modifiers including, but not limited to, gum arabic, maltodextrins, corn syrup, gelatin, cellulose type materials like carboxymethyl cellulose or hydroxymethyl cellulose, starch and modified starches, vegetable gums like alginates, locust bean gum, guar gum, and gum tragacanth, insoluble carbonates like calcium carbonate or magnesium carbonate and talc. Antitack agents may also be added as panning modifiers which allow the use of a variety of carbohydrates and sugar alcohols to be used in the development of new panned or coated gum products.

The coating may include one or more flavors and, in various embodiments, includes one or more encapsulated enzymes prepared in accordance with the present invention. In various such embodiments, an encapsulated protease enzyme is present in the coating at a concentration of from about 1 wt% to about 15 wt%, or from about 2.5 wt% to about 10 wt%, based on the total weight of the coating.

The presence of a coating generally increases the time it takes the tablet or lozenge to dissolve in the mouth. In various embodiments, a slow dissolving tablet or lozenge is preferred in order to provide a sustained release rate of the active ingredients (e.g., the encapsulated enzyme) over a period of time of from about 3 to about 15 minutes, or from about 5 to about 10 minutes.

### D. Enzyme Activity

It is to be noted that typically the means of encapsulating the enzyme (e.g., process conditions, coating types, etc.) and/or the means of preparing the oral composition (e.g., process conditions, including temperature, moisture content, pH, etc.) of the components which come into contact with the encapsulated enzyme, and/or the resulting oral composition comprising the encapsulated enzyme, are controlled in a manner which optimizes enzyme activity (i.e., the activity of the enzyme to remove stains from a tooth surface). Stated another way, the various process steps, conditions, composition components, etc. will typically be selected in order to ensure the enzyme remains substantially active, both after encapsulation and after inclusion in the oral composition. For example, the enzyme, after encapsulation and/or inclusion in the oral composition, may typically be at least about 25% active, at least about 50% active, at least about 75% active, at least about 85% active, at least about 95% active or more.

The present invention is further illustrated by the following Examples. These Examples are not to be regarded as limiting the scope of the invention or the manner in which it may be practiced.

### EXAMPLES

### Spray dried, fluid-bed coated, and spray chilled enzymes:

### Example 1

An encapsulated subtilisin enzyme is obtained having a content of 80 wt% shellac and 20 wt% subtilisin, by spray drying an alcohol/shellac/subtilisin solution, in accordance with encapsulation methods and techniques generally known in the art.

### Example 2

An encapsulated chymotrypsin enzyme is obtained having a content of 70 wt% Zein and 30 wt% chymotrypsin, by spray drying an alcohol/Zein/chymotrypsin solution having a solids content of 10 wt%.

### Example 3

An encapsulated trypsin enzyme is obtained having a content of 60 wt% shellac and 40 wt% trypsin, by fluid-bed coating trypsin with an alcohol/shellac solution having a solids content of 30 wt%.

### Example 4

An encapsulated subtilisin enzyme is obtained having a content of 85 wt% wax and 15 wt% subtilisin, by spray chilling a mixture of molten wax and subtilisin.

### Example 5

Am encapsulated pepsin enzyme is obtained having a content of 20 wt% Zein, 20 wt% shellac, and 60 wt% pepsin, by spray drying an alcohol/shellac/pepsin mixture and then fluid-bed coating the spray dried product to provide a second coating of alcohol and Zein.

### Example 6

An encapsulated subtilisin enzyme is obtained having a content of 30 wt% hydroxypropylmethyl cellulose (HPMC) and 70 wt% subtilisin, by fluid-bed coating subtilisin with an aqueous solution of HPMC having a solids content of 10 wet%.

### Example 7

An encapsulated subtilisin enzyme is obtained having a content of 40 wt% gum Arabic and 60 wt% subtilisin, by fluid-bed coating subtilisin with an aqueous solution of gum arabic having a solids content of 30 wt%.

### Agglomerated enzymes:

### Example 8

An agglomerated trypsin enzyme is obtained having a content of 15 wt% hydroxypropylmethyl cellulose (HPMC), and 85 wt% trypsin, by blending trypsin and HPMC, adding water to the mixture, and drying and grinding the resulting product.

### Example 9

An agglomerated rennin enzyme is obtained having a content of 15 wt% gelatin and 85 wt% rennin, by blending rennin and gelatin, adding water to the mixture, and drying and grinding the resulting product.

### Example 10

An agglomerated papain enzyme is obtained having a content of 10 wt% Zein and 90 wt% papain, by mixing papain with an alcohol solution containing 25% Zein, and drying and grinding the resulting product.

### Example 11

An agglomerated subtilisin enzyme is obtained having a content of 15 wt% maltodextrin and 85 wt% subtilisin, by mixing subtilisin and maltodextrin, then adding water, drying and grinding the resulting product.

### Agglomerated/encapsulated enzymes prepared by multi-step procedures:

### Example 12

Subtilisin is spray dried with maltodextrin to provide spray dried/encapsulated subtilisin having a solids content of 30 wt%. This powder is agglomerated with a hydroxypropylmethyl cellulose (HPMC) in a weight ratio (wt/wt) of 85/15 powder/HPMC, wetted with water and dried. After grinding, the resulting powder will contain about 68 wt% subtilisin, about 17 wt% maltodextrin, and about 15 wt% HPMC.

### Example 13

Trypsin is agglomerated with HPMC in a wt/wt ratio of 85/15 trypsin/HPMC. After drying and grinding, the resulting powder is fluid-bed coated with an alcohol/shellac solution at about 25% solids content to provide a final product containing about 60 wt% trypsin, about 10 wt% HPMC, and about 30 wt% shellac.

### Example 14

Chymotrypsin is agglomerated with HPMC in a wt/wt ratio of 85/15 chymotrypsin/HPMC. After drying and grinding, the resulting powder is agglomerated with a 15% solids, high-pH, aqueous solution of Zein to give a final product containing about 60% chymotrypsin, about 10% HPMC, and about 30% Zein.

### Example 15

Pepsin is spray dried with a 25 wt% solution of gelatin. The spray dried product is then agglomerated with a 15% solids, high-pH, aqueous solution of Zein. The final product will contain about 50% pepsin, about 20% gelatin, and about 30% Zein.

### Encapsulated enzymes prepared using absorption techniques:

### Example 16

A 10 wt% solution of bromelain is sprayed onto a microcrystalline cellulose powder. The resulting mixture is dried and ground to provide a product that is about 70 wt% microcrystalline cellulose and about 30 wt% bromelain.

### Example 17

A 10 wt% solution of subtilisin is sprayed onto a high absorption starch and the resulting mixture is dried and ground to provide a product that is about 80 wt% starch and about 20 wt% subtilisin.

Encapsulated enzymes prepared using absorption techniques including drying the enzyme together with a sugar or sugar alcohol, and/or resolidifying the enzyme with a sugar or sugar alcohol when mixed together in a molten state:

### Example 18

Papain is added to molten sorbitol in a weight ratio of 90 parts sorbitol to 10 parts papain. After mixing, the blend is cooled and ground.

### Example 19

A mixture containing 4 wt% trypsin dissolved in 96 wt% high fructose corn syrup is prepared. The mixture is then evaporated to low moisture and ground.

The products of examples 1-19 are suitable for incorporation into the formulations shown in Tables 1-5.

### Examples 20-24

Typical chewing gum formulations prepared in accordance with the present invention are set forth in Table 1. Prepare the chewing gums and encapsulate the enzyme as detailed herein. Suitable flavors and coolants are generally known in the art. Values are % by weight of the chewing gum composition or encapsulated enzyme.

**Table 1**

| Ingredient | Example 20 | Example 21 | Example 22 | Example 23 | Example 24 |
|---|---|---|---|---|---|
| Gum Base | 34.27 | 26.22 | 41.00 | 42.00 | 32.72 |
| Lecithin | 0.17 | 0.17 | 0.17 | 0.17 | 0.05 |
| Sorbitol | 50.06 | 49.86 | 30.00 | 35.00 | |
| Xylitol | | | | | 60.00 |
| Maltitol | | | 4.00 | 15.00 | |
| Calcium Carbonate | 10.00 | 17.5 | 20.33 | 1.08 | |
| Glycerin | | 4.00 | 1.00 | | |
| Sorbitol syrup | 2.50 | | | 4.50 | |
| Corn syrup | | | | | 5.0 |
| High intensity sweetener | 0.60 | 0.60 | 0.60 | 0.30 | 0.30 |
| Encapsulated high intensity sweetener | | | | 0.30 | 0.30 |
| Coolant | 0.30 | | 0.30 | | |
| Encapsulated subtilisin (30 wt% enzyme based on wt. of encapsulated enzyme) | 0.50 | 0.05 | 1.00 | 0.025 | 0.03 |
| Encapsulated chymotrypsin (20 wt% enzyme based on wt. of encapsulated enzyme) | | | | 0.025 | |
| Encapsulated trypsin (10 wt% enzyme based on wt. of encapsulated enzyme) | | | | 0.025 | |
| Flavor | 1.60 | 1.60 | 1.60 | 1.60 | 1.60 |
| TOTAL | 100.00% | 100.00% | 100.00% | 100.00% | 100.00% |

### Example 25

A tableted/compressed chewing gum was prepared as detailed herein, including incorporating an encapsulated enzyme (i.e., subtilisin) prepared by spray drying. The formulation is set forth below in Table 2. Values are % by weight of the chewing gum composition or encapsulated enzyme.

**Table 2**

| Ingredient | Example 25 |
|---|---|
| Gum Base | 84.30 |
| Silicon dioxide | 0.50 |
| Magnesium stearate | 1.50 |
| Encapsulated high intensity sweetener | 0.50 |
| Encapsulated subtilisin (25 wt% enzyme based on wt. of encapsulated enzyme) | 10.00 |
| Menthol | 0.20 |
| Spray dried flavor | 3.00 |
| TOTAL | 100.00% |

### Example 26-34

Typical tableted/compressed chewing gums are prepared in accordance with the present invention as set forth below in Tables 3 and 4. Prepare the tableted/compressed chewing gums and encapsulate the enzyme as detailed herein. Values are % by weight of the chewing gum composition or encapsulated enzyme.

**Table 3**

| Ingredient | Example 26 | Example 27 | Example 28 | Example 29 |
|---|---|---|---|---|
| Gum Base | 87.93 | 75.03 | 80.20 | 92.50 |
| Powdered sugar | | 10.00 | | |
| Sorbitol | | | 5.00 | |
| Color | 0.05 | 0.02 | | |
| Silicon dioxide | 1.00 | 1.00 | 1.00 | 1.00 |
| Magnesium stearate | 0.50 | 0.90 | 0.50 | 0.50 |
| Encapsulated high intensity sweetener | 0.52 | 0.55 | 0.55 | 1.00 |
| Encapsulated subtilisin (25 wt% enzyme based on wt. of encapsulated enzyme) | | 5.00 | | |
| Encapsulated trypsin (40 wt% enzyme based on wt. of encapsulated enzyme) | 5.00 | 2.50 | | |
| Encapsulated subtilisin (30 wt% enzyme based on wt. of encapsulated enzyme) | | | 7.55 | 2.00 |
| Menthol | | | 0.20 | |
| Spray dried flavor | 5.00 | 5.00 | 5.00 | 3.00 |
| TOTAL | 100.00% | 100.00% | 100.00% | 100.00% |

**Table 4**

| Ingredient | Example 30 | Example 31 | Example 32 | Example 33 | Example 34 |
|---|---|---|---|---|---|
| Gum Base | 75.00 | 36.17 | 55.00 | 80.20 | 92.50 |
| Dextrin | 13.25 | | | 2.00 | |
| Xylitol | | | 35.23 | | |
| Sorbitol | | 55.75 | | 8.97 | 1.00 |
| Color | 1.00 | 0.50 | 0.50 | | 0.50 |
| Silicon dioxide | 0.25 | 0.56 | | | |
| Magnesium stearate | 0.25 | | 0.50 | 0.08 | 0.50 |
| High intensity sweetener | 0.25 | 0.02 | 0.55 | 0.50 | |
| Encapsulated high intensity sweetener | | 1.00 | | 0.50 | 4.50 |
| Encapsulated subtilisin (20 wt% enzyme based on wt. of encapsulated enzyme) | 4.50 | | 7.00 | 7.75 | |
| Encapsulated trypsin (40 wt% enzyme based on wt. of encapsulated enzyme) | | | 1.00 | | |
| Encapsulated subtilisin (30 wt% enzyme based on wt. of encapsulated enzyme) | 5.00 | 5.00 | | | 1.00 |
| Spray dried flavor | 0.50 | 1.00 | 0.22 | | |
| TOTAL | 100.00% | 100.00% | 100.00% | 100.00% | 100.00% |

### Examples 35-39

Typical tablet compositions are prepared in accordance with the present invention as set forth below in Table 5. Prepare the tablets and encapsulate the enzyme as detailed herein. Suitable flavors are those generally known in the art. Values are % by weight of the tablet or encapsulated enzyme.

**Table 5**

| Ingredient | Example 35 | Example 36 | Example 37 | Example 38 | Example 39 |
|---|---|---|---|---|---|
| Sorbitol | 94.08 | | 94.44 | 56.64 | 90.08 |
| Color | | 0.05 | 0.02 | 0.02 | 0.20 |
| Xylitol | | 93.30 | | 35.30 | |
| Silicon dioxide | | | 0.19 | 0.19 | |
| Magnesium stearate | 1.01 | 0.50 | 1.01 | 1.01 | 0.50 |
| High intensity sweetener | 0.58 | 0.39 | 0.39 | 0.39 | 0.19 |
| Encapsulated high intensity sweetener | | | 0.19 | 0.19 | 0.39 |
| Encapsulated subtilisin (20 wt% enzyme based on wt. of encapsulated enzyme) | 0.57 | | 2.50 | 5.50 | |
| Encapsulated trypsin (40 wt% enzyme based on wt. of encapsulated enzyme) | 3.00 | | | | |
| Encapsulated subtilisin (30 wt% enzyme based on wt. of encapsulated enzyme) | | 5.00 | 0.50 | | 7.88 |
| Coolant | | | | | |
| Flavor | 0.76 | 0.76 | 0.76 | 0.76 | 0.76 |
| TOTAL | 100.00% | 100.00% | 100.00% | 100.00% | 100.00% |

### Example 40

A typical foam composition is prepared in accordance with the present invention as set forth below in Table 6, including lyophilized (i.e., freeze-dried) subtilisin that is prepared as described herein. Suitable flavors and cooling agents are those generally known in the art. Values are % by weight of the foam.

**Table 6**

| Ingredient | Example 40 |
|---|---|
| Menthol | 0.2% |
| Cooling agent | 0.36% |
| High Intensity Sweetener | 0.31% |
| Flavor | 1.00% |
| Water | 85.63% |
| Lyophilized subtilisin | 12.00% |
| Sodium lauryl sulfate | 0.50% |
| TOTAL | 100.00% |

### Example 41

This example describes encapsulating subtilisin in acacia by spray drying.

A spray drying solution was prepared by mixing an acacia solution and a subtilisin solution (4:1 weight ratio of acacia solution to subtilisin solution) in water. The subtilisin solution contained 8 wt% subtilisin and an 80:20 (w/w) mixture of glycerin and water. The acacia solution included 80 wt% acacia dissolved in water. The spray drying solution exhibited a Brix value (determined using means known in the art) of approximately 29%.

The spray drying solution was introduced to a spray dryer including an atomizer wheel and commercially available from Spray-Tech (Oklahoma City, OK) to encapsulate subtilisin within acacia as detailed herein. The air temperature at the dryer inlet was approximately 180°C, and the air temperature of the dryer outlet was approximately 80°C. The spray drying solution was introduced to the spray dryer utilizing a pump operated at a rate of approximately 35-37 revolutions per minute (rpm).

The resulting product contained approximately 2.6 wt% subtilisin.

### Example 42 (Background Example)

This example details in-vitro stain removal screening of subtilisin.

Bovine teeth samples were cut to prepare an enamel surface of approximately 10 mm². The teeth surfaces were polished with sandpaper and slightly etched with acid to facilitate greater stain adhesion.

Each surface was then treated over the course of 4 days by alternating cycles of contact with air and contacted with a staining broth (at approximately 37°C); the air-broth cycle was repeated twice each day.

The broth was a mixture of finely ground instant coffee (Maxwell House®) and tea (Lipton®) (at a weight ratio 1:1), 24-hour sarcina lutea turtox culture (approximately 10:1 volume to weight ratio of culture to coffee or tea) and gastric mucin commercially available from Sigma-Aldrich (0.75:1 weight ratio of gastric mucin to coffee or tea) in a sufficient quantity of sterilized trypticase soy solution, also commercially available from Sigma-Aldrich. After staining, the enamel surfaces were rinsed with deionized water, dried overnight, and the stain intensity measured using a Minolta CM 2600-d spectrophotometer (i.e., L*, a*, b*, and E values were obtained).

Solutions were prepared consisting of sodium chloride (approximately 10 wt%), sodium bicarbonate (approximately 86 wt%), and sodium carbonate (approximately 4 wt%), each commercially available from Spectrum Chemical, to which the stain removal active (commercially available subtilisin) was added. The bovine teeth surface samples (N = 8) were then treated in these solutions twice daily (each time for a period of ten minutes) for a total of 7 days. The surfaces were stored overnight during the testing in humid saliva film.

After 4 days and 7 days of treatment, the stain intensity for each surface using the spectrophotometer described above, and the stain removal efficacies were calculated based on the change in E values, dE, as measured before and after treatment of the samples.

For purposes of comparison, subtilisin efficacy was compared to that of a negative control not believed to exhibit substantial stain removal efficacy (artificial saliva) and a positive control reported as exhibiting stain removal efficacy (Na-hexametaphosphate).

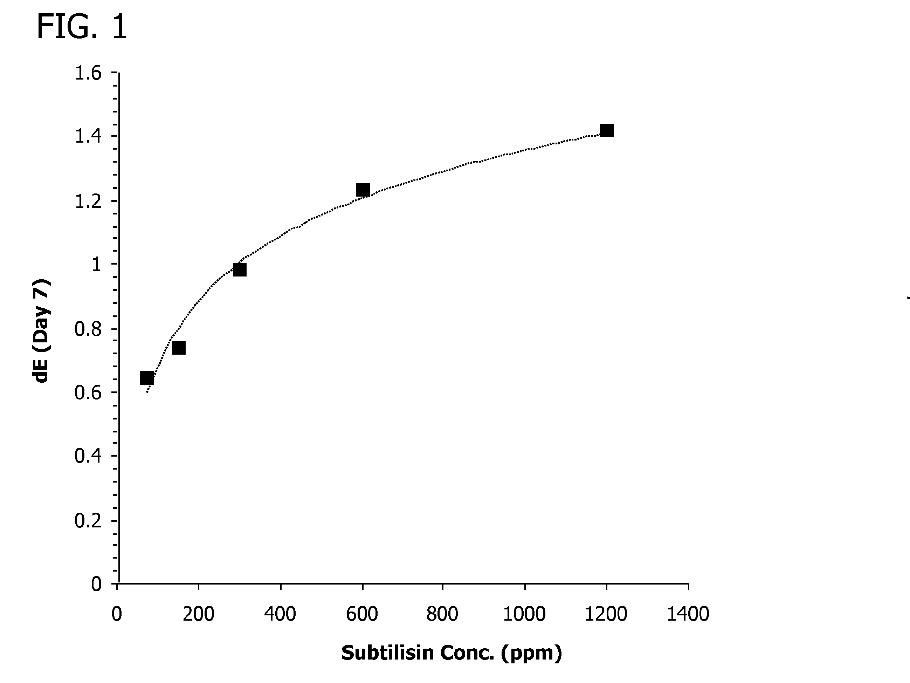

As shown in Fig. 1, subtilisin provided a logarithmic stain removal dose response (y = 0.2938Ln(x) - 0.6716; R² = 0.9831) in the range of active concentration from 75 parts per million (ppm) to 1200 ppm, and was shown to be statistically significant (p < 0.05) based on regression analysis.

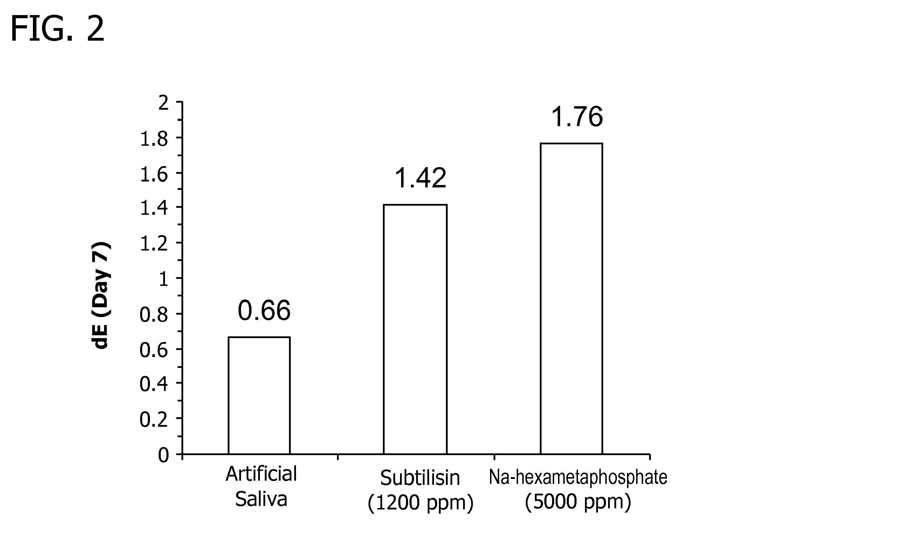

As shown in Fig. 2, at concentrations greater than or equal to 1200 ppm, subtilisin also demonstrated significantly higher stain removal efficacy than the artificial saliva control and an equivalent efficacy to positive control Na-hexametaphosphate at 5000 ppm in solution (p < 0.05).

The concentrations of subtilisin and positive control Na-hexametaphosphate were selected to approximate the amount of active that would be released from a chewing gum during a typical 20-minute chew by a consumer.

### Example 43

This example describes the release profile for an encapsulated active from compressed chewing gums prepared as detailed herein during chew-out testing. Encapsulated enzyme constituted 6 wt% of each 2 pellet chewing gum serving.

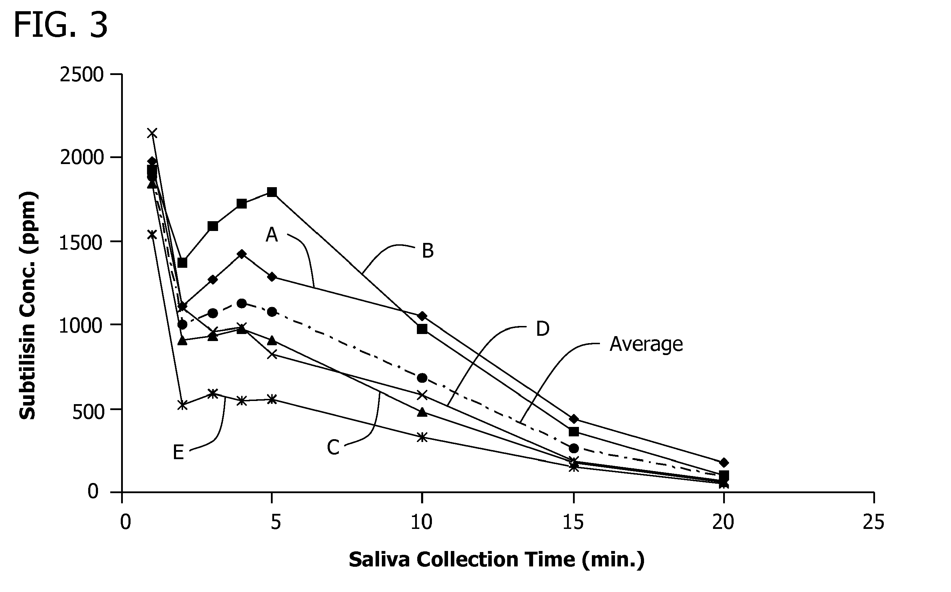

Chewing gum was chewed by 5 panelists (A-E) for a total time of 20 minutes. Saliva was collected from each of the panelists at time intervals of 1, 2, 3, 4, 5, 10, 15, and 20 minutes. A plot of the concentrations of active (i.e., subtilisin) in the saliva samples versus sample time is shown in Fig. 3.

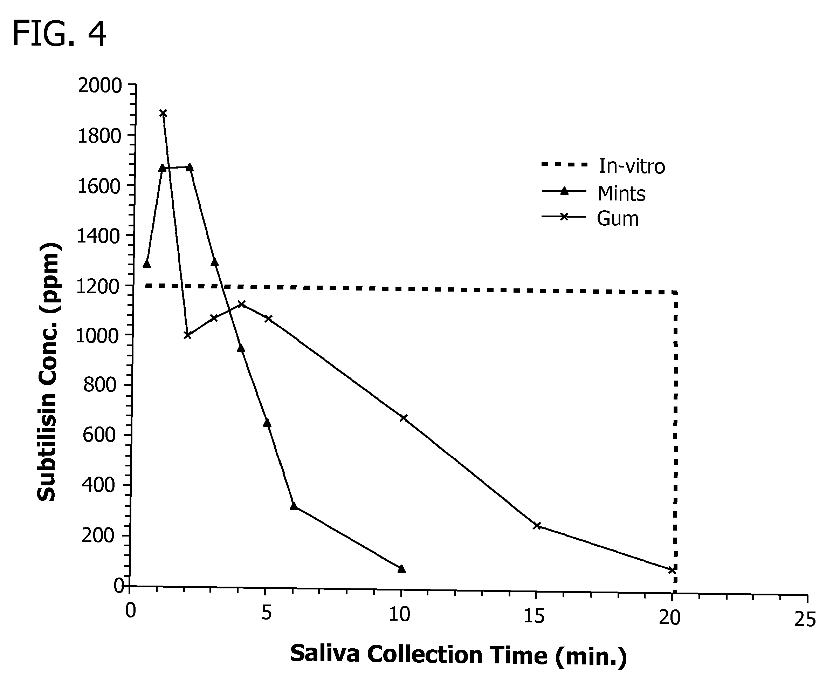

Fig. 4 provides a comparison of in-vitro active testing described in Example 42 and daily active dose estimates prepared in view of the chewing gum testing detailed in this example.

Specifically, in the in-vitro testing of Example 42, bovine enamel was exposed to 1200 ppm of subtilisin for 20 minutes daily (i.e., an exposure of 24,000 ppm-minutes daily). This corresponds to the area under the in-vitro testing curve in Fig. 4.

Fig. 4 also includes a Gum curve corresponding to the average subtilisin concentration curve from Fig. 3. This curve represents a subtilisin dose of 13,530 ppm during the 20 minutes of chew-out tests (i.e., the area under the Gum curve). Assuming 4 daily servings, chewing gums of the type tested are believed to provide a daily active dose of approximately 54,120 ppm-minutes. Notably, this active dose is more than twice that of the daily subtilisin dose utilized in the in-vitro testing which, as demonstrated in Example 42, provided stain removal efficacy greater than the negative control and at least equivalent to that of the positive control.

Fig. 4 also includes a curve for mint testing which corresponds to a daily subtilisin dose of approximately 26,000 ppm-minutes.

The present invention is not limited to the above embodiments and can be variously modified. The above description of the preferred embodiments, including the Examples, is intended only to acquaint others skilled in the art with the invention, its principles, and its practical application so that others skilled in the art may adapt and apply the invention in its numerous forms, as may be best suited to the requirements of a particular use.

With reference to the use of the word(s) comprise or comprises or comprising in this entire specification (including the claims below), unless the context requires otherwise, those words are used on the basis and clear understanding that they are to be interpreted inclusively, rather than exclusively, and applicants intend each of those words to be so interpreted in construing this entire specification.

## Claims

1. An oral composition effective for stain removal from a tooth surface, the composition comprising:
an encapsulated protease enzyme having a moisture content of less than 2 wt %, wherein the encapsulated protease enzyme comprises a protease enzyme effective for stain removal from a tooth surface and an encapsulant at least partially coating a surface of the protease enzyme.

2. The oral composition of claim 1, wherein the protease enzyme is selected from the group consisting of cathepsins, pepsin, rennin, thermolysin, trypsin, elastase, chymotrypsin, papain, bromelain, subtilisin, and combinations thereof.

3. The oral composition of claim 1, wherein the encapsulated protease enzyme constitutes from about 0.02 wt% to about 10 wt% of the oral composition.

4. The oral composition of claim 1, wherein the protease enzyme constitutes from about 10 wt% to about 50 wt% of the encapsulated protease enzyme.

5. The oral composition of claim 1, wherein the encapsulated protease enzyme comprises a protease enzyme and an encapsulant substantially coating all the enzyme surface.

6. The oral composition of claim 1, wherein the oral composition is in the form of a chewing gum, confection, lozenge, pressed tablet, edible film, mouthspray, dentrifice, mouthwash, foam, toothpaste product, or a combination thereof.

7. The oral composition of claim 1, wherein the oral composition further comprises a coating on the surface of the oral composition.

8. A method for preparing an oral composition effective for stain removal from a tooth surface, the method comprising:
contacting an encapsulated protease enzyme with one or more components of the oral composition, wherein the encapsulated protease enzyme having a moisture content of less than 2 wt % comprises a protease enzyme effective for stain removal from a tooth surface and an encapsulant at least partially coating a surface of the enzyme.

9. The method of claim 8, wherein contacting the encapsulated enzyme and the one or more components of the oral composition comprises:
placing the one or more components in a vessel, wherein the one or more components are selected from a sweetener, gum base, flavor, polyol, or a combination thereof;
placing the encapsulated enzyme in the vessel; and
mixing the contents of the vessel to contact the one or more components and the encapsulated enzyme.

10. The method of claim 9, wherein the method further comprises forming the encapsulated protease enzyme by one or more processes selected from the group consisting of spray drying, spray cooling, spray chilling, extrusion, coacervation, molecular inclusion, fluid bed coating, granulation, agglomeration, roll compaction, and combinations thereof.

11. The method of claim 8, wherein the protease enzyme is subtilisin.

12. The method of claim 8, wherein the encapsulated protease enzyme comprises a protease enzyme and an encapsulant substantially coating all of the enzyme particle surface.

13. The method of claim 8, wherein the encapsulant is selected from the group consisting of acrylic polymers and copolymers, carboxyvinyl polymer, polyamides, polystyrene, polyvinyl acetate, polyvinyl acetate phthalate, polyvinylpyrrolidone, waxes, and combinations thereof.

14. The method of claim 8, wherein the oral composition is in the form of either (i) a compressed chewing gum and the one or more components of the oral composition comprise a powdered gum base, or alternatively (ii) the oral composition is in the form of a pressed tablet and the one or more components of the oral composition comprise a polyol selected from the group consisting of mannitol, xylitol, sorbitol, maltitol, hydrogenated isomaltalose, lactitol, erythritol, and combinations thereof.

## Patentansprüche

1. Orale Zusammensetzung, wirksam für Fleckenentfernung von einer Zahnoberfläche, die Zusammensetzung umfassend
ein eingekapseltes Protease-Enzym mit einem Feuchtigkeitsgehalt von geringer als 2 Gew.-%, wobei das eingekapselte Protease-Enzym ein Protease-Enzym umfasst, wirksam für Fleckenentfernung von einer Zahnoberfläche, und ein Verkapselungsmittel, das mindestens teilweise eine Oberfläche des Protease-Enzyms bedeckt.

2. Orale Zusammensetzung gemäß Anspruch 1, wobei das Protease-Enzym ausgewählt ist aus der Gruppe Cathepsine, Pepsin, Rennin, Thermolysin, Trypsin, Elastase, Chymotrypsin, Papain, Bromelain, Subtilisin und deren Gemische.

3. Orale Zusammensetzung gemäß Anspruch 1, wobei das eingekapselte Protease-Enzym von etwa 0,02 Gew.-% bis etwa 10 Gew.-% der oralen Zusammensetzung ausmacht.

4. Orale Zusammensetzung gemäß Anspruch 1, wobei das Protease-Enzym von etwa 10 Gew.-% bis etwa 50 Gew.-% des eingekapselten Protease-Enzyms ausmacht.

5. Orale Zusammensetzung gemäß Anspruch 1, wobei das eingekapselte Protease-Enzym ein Protease-Enzym umfasst und ein Verkapselungsmittel, das im Wesentlichen die gesamte Enzymoberfläche bedeckt.

6. Orale Zusammensetzung gemäß Anspruch 1, wobei die orale Zusammensetzung in der Form eines Kaugummis, eines Konfekts, einer Pastille, einer Presstablette, eines essbaren Films, eines Mundsprays, eines Zahnputzmittels, eines Mundwassers, eines Schaums, eines Zahnpastaprodukts oder einer Kombination davon ist.

7. Orale Zusammensetzung gemäß Anspruch 1, wobei die orale Zusammensetzung zudem eine Beschichtung auf der Oberfläche der oralen Zusammensetzung umfasst.

8. Herstellungsverfahren für eine orale Zusammensetzung, wirksam für Fleckenentfernung von einer Zahnoberfläche, das Verfahren umfassend
Zusammenbringen eines eingekapselten Protease-Enzyms mit einem oder mehreren Bestandteilen der oralen Zusammensetzung, wobei das eingekapselte Protease-Enzym mit einem Feuchtigkeitsgehalt von geringer als 2 Gew.-% ein Protease-Enzym umfasst, wirksam für Fleckenentfernung von einer Zahnoberfläche, und ein Verkapselungsmittel, das mindestens teilweise eine Oberfläche des Enzyms bedeckt.

9. Verfahren gemäß Anspruch 8, wobei Zusammenbringen des eingekapselten Protease-Enzyms mit dem einen oder den mehreren Bestandteilen der oralen Zusammensetzung umfasst
Platzieren des einen oder der mehreren Bestandteile in ein Gefäß, wobei der eine oder die mehreren Bestandteile ausgewählt sind aus einem Süßstoff, einer Kaumasse, einem Geschmack, einem Polyol oder einer Kombination davon;
Platzieren des eingekapselten Enzyms in das Gefäß; und
Vermischen des Inhalts des Gefäßes zum Zusammenbringen des einen oder der mehreren Bestandteile und des eingekapselten Enzyms.

10. Verfahren gemäß Anspruch 9, wobei das Verfahren zudem umfasst Bilden des eingekapselten Protease-Enzyms durch ein oder mehrere Verfahren, ausgewählt aus der Gruppe Spraytrocknung, Spraykühlung, Spraygefrierung, Extrusion, Coacervation, molekulare Inklusion, Fluidbettbeschichtung, Granulierung, Agglomerierung, Rollenkompaktierung und Kombinationen davon.

11. Verfahren gemäß Anspruch 8, wobei das Protease-Enzym Subtilisin ist.

12. Verfahren gemäß Anspruch 8, wobei das eingekapselte Protease-Enzym ein Protease-Enzym umfasst und ein Verkapselungsmittel, das im Wesentlichen die gesamte Kornoberfläche des Enzyms beschichtet.

13. Verfahren gemäß Anspruch 8, wobei das Verkapselungsmittel ausgewählt ist aus der Gruppe Acrylpolymere und -copolymere, Carboxyvinylpolymer, Polyamide, Polystyrol, Polyvinylacetat, Polyvinylacetatphthalat, Polyvinylpyrrolidon, Wachse und Kombinationen davon.

14. Verfahren gemäß Anspruch 8, wobei dioe orale Zusammensetzung in der Form ist, entweder (i) eines komprimierten Kaugummis und der eine oder die mehreren Bestandteile der oralen Zusammensetzung eine pulverisierte Kaumasse umfassen, oder alternativ (ii) die orale Zusammensetzung in der Form ist einer Presstablette und der eine oder die mehreren Bestandteile der oralen Zusammensetzung umfassen ein Polyol, ausgewählt aus der Gruppe Mannitol, Xylitol, Sorbitol, Maltitol, hydrogenierte Isomaltalose, Lactitol, Erythritol und Kombinationen davon.

## Revendications

1. Une composition orale efficace pour l'élimination de taches d'une surface dentaire, la composition contenant :
une enzyme protéase encapsulée possédant une teneur en humidité inférieure à 2 % en poids, où l'enzyme protéase encapsulée comprend une enzyme protéase efficace pour l'élimination de taches d'une surface dentaire et un encapsulant recouvrant au moins partiellement une surface de l'enzyme protéase.

2. La composition orale selon la revendication 1, où l'enzyme protéase est sélectionnée dans le groupe se composant de cathepsines, pepsine, rennine, thermolysine, trypsine, élastase, chymotrypsine, papaïne, bromélaïne, subtilisine, et des combinaisons de ceux-ci.

3. La composition orale selon la revendication 1, où l'enzyme protéase encapsulée constitue d'environ 0,02 % en poids à environ 10 % en poids de la composition orale.

4. La composition orale selon la revendication 1, où l'enzyme protéase constitue d'environ 10 % en poids à environ 50 % en poids de l'enzyme protéase encapsulée.

5. La composition orale selon la revendication 1, où l'enzyme protéase encapsulée comprend une enzyme protéase et un encapsulant recouvrant sensiblement la totalité de la surface de l'enzyme.

6. La composition orale selon la revendication 1, où la composition orale se présente sous la forme d'un chewing-gum, d'une confiserie, d'une pastille, d'un comprimé, d'un film consommable, d'un aérosol buccal, d'un dentifrice, d'un bain de bouche, d'une mousse, d'un produit de pâte dentifrice, ou d'une combinaison de ceux-ci.

7. La composition orale selon la revendication 1, où la composition orale comprend en outre un revêtement sur la surface de la composition orale.

8. Un procédé de préparation d'une composition orale efficace pour l'élimination de taches d'une surface dentaire, le procédé comprenant :
la mise en contact d'une enzyme protéase encapsulée avec un ou plusieurs composants de la composition orale, où l'enzyme protéase encapsulée possédant une teneur en humidité inférieure à 2 % en poids contient une enzyme protéase efficace pour l'élimination de taches d'une surface dentaire et un encapsulant recouvrant au moins partiellement une surface de l'enzyme.

9. Le procédé selon la revendication 8, où la mise en contact de l'enzyme encapsulée et des un ou plusieurs composants de la composition orale comprend :
le placement des un ou plusieurs composants dans un récipient, où les un ou plusieurs composants sont sélectionnés parmi un édulcorant, une base de gomme, un arôme, un polyol, ou une combinaison de ceux-ci,
le placement de l'enzyme encapsulée dans le récipient, et
le mélange du contenu du récipient de façon à mettre en contact les un ou plusieurs composants et l'enzyme encapsulée.

10. Le procédé selon la revendication 9, où le procédé comprend en outre la formation de l'enzyme protéase encapsulée par un ou plusieurs procédés sélectionnés dans le groupe se composant de séchage par pulvérisation, refroidissement par pulvérisation, rafraîchissement par pulvérisation, extrusion, coacervation, inclusion moléculaire, revêtement à lit fluidisé, granulation, agglomération, compactage au rouleau, et des combinaisons de ceux-ci.

11. Le procédé selon la revendication 8, où l'enzyme protéase est subtilisine.

12. Le procédé selon la revendication 8, où l'enzyme protéase encapsulée contient une enzyme protéase et un encapsulant recouvrant sensiblement la totalité de la surface particulaire de l'enzyme.

13. Le procédé selon la revendication 8, où l'encapsulant est sélectionné dans le groupe se composant de polymères et copolymères acrylique, polymère de carboxyvinyle, polyamides, polystyrène, acétate de polyvinyle, phthalate d'acétate de polyvinyle, polyvinylpyrrolidone, cires, et des combinaisons de ceux-ci.

14. Le procédé selon la revendication 8, où la composition orale se présente sous la forme de soit (i) un chewing-gum comprimé, et les un ou plusieurs composants de la composition orale contiennent une base de gomme en poudre, ou dans une variante (ii) la composition orale se présente sous la forme d'un comprimé et les un ou plusieurs composants de la composition orale contiennent un polyol sélectionné dans le groupe se composant de mannitol, xylitol, sorbitol, maltitol, isomaltalose hydrogénée, lactitol, érythritol, et des combinaisons de ceux-ci.
